# EUROPEAN PATENT SPECIFICATION

(11) **EP 3 962 559 B1**
(45) Date of publication and mention of the grant of the patent: **16.04.2025**
(21) Application number: 20795069.2
(22) Date of filing: 24.04.2020
(51) Int. Cl.: A61L 29/16, A61L 31/16, C12N 5/095, A61P 1/00, A61K 31/351, A61K 31/407, A61K 31/427, A61K 31/675, A61K 31/69, A61K 45/06

(54) **METHODS AND COMPOSITIONS FOR TREATING CHRONIC INFLAMMATORY INJURY, METAPLASIA, DYSPLASIA AND CANCERS OF EPITHELIAL TISSUES**
VERFAHREN UND ZUSAMMENSETZUNGEN ZUR BEHANDLUNG VON CHRONISCHEN ENTZÜNDUNGSVERLETZUNGEN, METAPLASIE, DYSPLASIE UND KARZINOMEN DES EPITHELGEWEBES
MÉTHODES ET COMPOSITIONS POUR TRAITER UNE LÉSION INFLAMMATOIRE CHRONIQUE, UNE MÉTAPLASIE, UNE DYSPLASIE ET DES CANCERS DES TISSUS ÉPITHÉLIAUX

(30) Priority: 26.04.2019 US 201962839152 P; 23.10.2019 US 201962924978 P
(43) Date of publication of application: 09.03.2022
(73) Proprietor: University of Houston System, Houston, TX 77204-2015 (US); The Board Of Regents Of The University Of Texas System, Austin, TX 78701 (US); Tract Pharmaceuticals, Inc., West Hartford, CT 06107 (US)
(72) Inventor: MCKEON, Frank, Sugar Land, TX 77479 (US); DULEBA, Marcin, Houston, TX 77003 (US); ZHANG, Yanting, Houston, TX 77025 (US); XIE, Jingzhong, Houston, TX 77025 (US); XIAN, Wa, Sugar Land, TX 77479 (US); VINCENT, Matthew, Amesbury, MA 01913 (US)
(74) Representative: Potter Clarkson
(86) International application number: PCT/US2020/029933
(87) International publication number: WO 2020/219963

(56) References cited:
- WO-A1-2013/071264
- WO-A1-2015/158809
- WO-A1-2017/190009
- WO-A1-2018/204913
- US-A1- 2014 255 430
- US-A1- 2016 022 976
- US-B2- 9 339 516

## Description

### Priority Claim

The present application claims benefit of priority to U.S. Provisional Applications Serial Nos. 62/839,152 and 62/924,978, filed April 26, 2019 and October 23, 2019, respectively.

### Federal Funding Disclosure

The invention was made with government support under Grant No. U24CA228550 awarded by the National Institutes of Health. The government has certain rights in the invention.

### Background

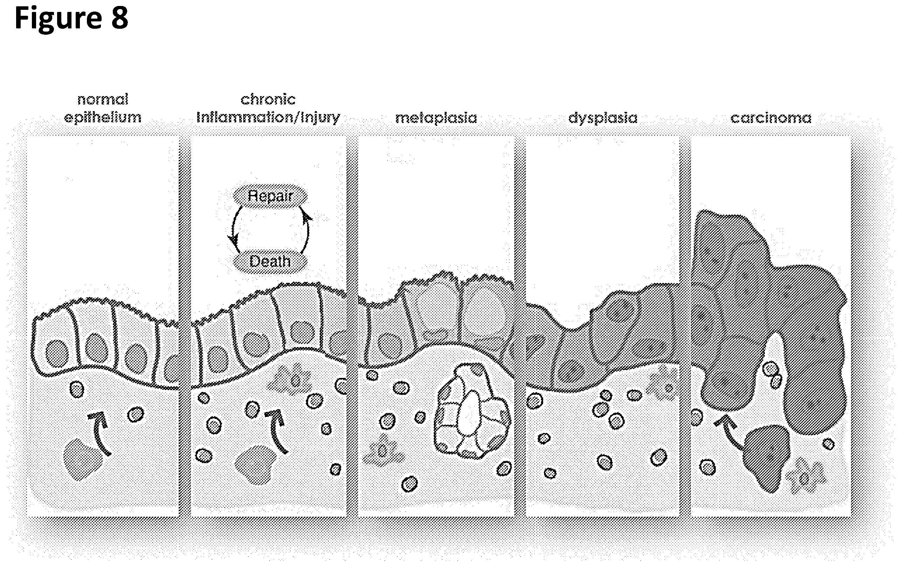

Metaplasia is the replacement of one differentiated cell type with another mature differentiated cell type that is not normally present in a specific tissue. Typically, metaplasia is triggered by environmental stimuli, which may act in concert with the deleterious effects of microorganisms and inflammation. A hallmark of metaplasia is a change in cellular identity. Universally, metaplasia is a precursor to low-grade dysplasia, which can culminate in high-grade dysplasia and carcinoma. See Figure 8. Typically, the risk of a patient developing cancer increases in a pronounced manner as an inflammatory disease or metaplasia progresses to dysplasia.

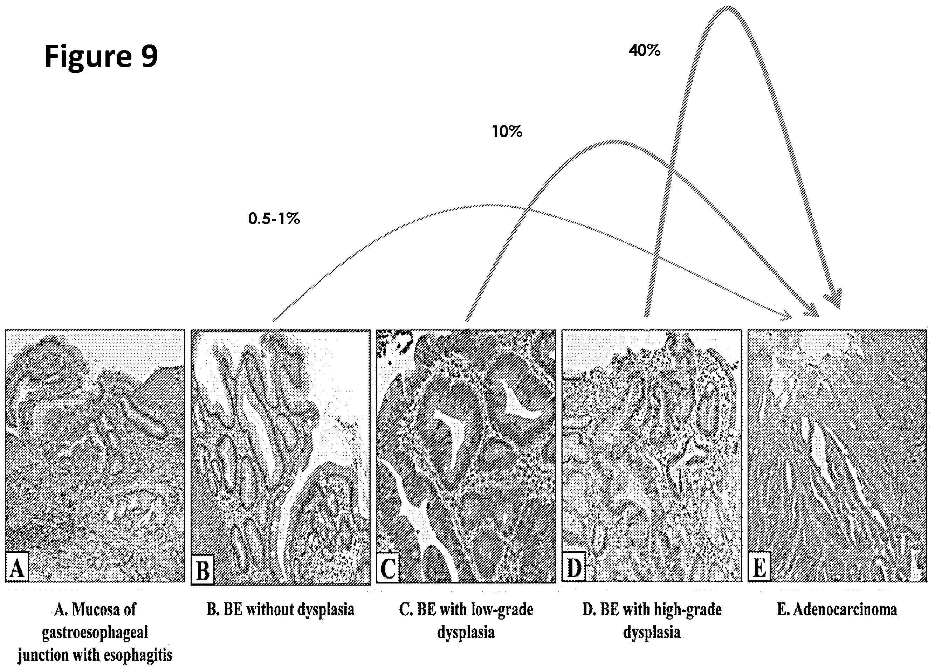

Figure 9 provides a statistical overview of the risk associated with Barret's Esophagus (BE). BE is the result of chronic gastroesophageal reflux disease (GERD) and represents the end stage of the natural course of this disease. It has been estimated that 20% of the population in the United States suffers from gastroesophageal reflux and that about 10% of these patients are diagnosed with BE. Commonly, BE is discovered during endoscopy for the evaluation of GERD symptoms.

It is documented that longstanding exposure of esophageal mucosa to gastric acidity results in cellular damage of the stratified squamous epithelium and creates an abnormal environment, which stimulates repair in the form of intestinal epithelial metaplasia. The consequence is that the stratified squamous epithelium, which physiologically lines the esophageal mucosa, is replaced by a pathological, specialized columnar epithelium which is neither of cardiac nor of stomach type, but exhibits features of the intestinal type of epithelium. This pathological type of epithelium usually demonstrates DNA alterations that predispose to malignancy. The alterations in BE are histologically classified into three categories, depending on whether or not they exhibit dysplasia: (1) BE without dysplasia; (2) BE with low-grade dysplasia; and (3) BE with high-grade dysplasia (HGD). In BE with HGD, dysplasia is confined to the mucosa without crossing the basement membrane. If dysplasia extends beyond the basement membrane into the lamina propria through the incoming lymphatic network, it is defined as intramucosal (superficial) adenocarcinoma, whereas if it invades the muscularis mucosa layer it becomes invasive adenocarcinoma. Thus, BE with HGD is considered a precursor of invasive adenocarcinoma.

Six to twenty percent of patients with BE and HGD are at greatest risk of developing adenocarcinoma within a short period of time, ranging from 17 to 35 month at follow-up. Esophagectomy specimens from patients with BE and HGD revealed invasive adenocarcinoma in 30%-40% of cases. A recent meta-analysis demonstrated that patients with BE and HGD developed esophageal adenocarcinoma with an average incidence of 6 every 100 patients per year, during the first 1.5 to 7 years of endoscopic surveillance. Furthermore, the majority of esophageal adenocarcinoma is thought to have evolved from cells that have undergone Barrett's metaplasia.

BE is also classified into two categories according to the extent of intestinal metaplasia above the gastroesophageal junction: (1) long segment BE, if the extent of the intestinal epithelium is greater than 3 cm; and (2) short segment BE, if it is less than 3 cm. Among patients who undergo endoscopy for symptoms of GERD, the incidence of long segment BE is 3%-5%, whereas short segment BE occurs in 10%-15%. Whether long and short segment BE share the same pathogenetic alterations or the same predisposition to malignancy still remains unclear; however, both conditions are currently treated in the same manner.

A common, and invasive, means for treating certain Barrett's Esophagus patients is through endoscopic ablation therapy, such as radiofrequency ablation, photodynamic therapy or cryoablation of esophageal tissue. However, despite a reasonably high percentage of patients that reach remission after therapy, many of those patients relapse within a few years. For other patients, whether because they are refractory to ablative therapy or ineligible due to severe co-morbidities, there are even fewer treatment options and those that exist still leave a significant need for more effective therapies with better results and/or long durations of remission.

Similar metaplasia-to-dysplasia-to-cancer transitions are observed across a variety of other epithelial tissues. Metaplasia tends to occur in tissues constantly exposed to environmental agents, which are often injurious in nature. For example, the pulmonary system (lungs and trachea) and the gastrointestinal tract are common sites of metaplasia owing to their contacts with air and food, respectively. In the ovaries, the dynamic interaction between ovarian surface epithelium and underlying ovarian stroma appears to be the origin of epithelial differentiation, metaplasia and finally malignant transformation.

There is a substantial unmet medical need not only for treatments that are effective for cancers of epithelial tissues, but also treatments directed to metaplasia and dysplasia of those tissues.

WO 2015/158809 describes a method of treating cancer from epithelial originin a patient, the method comprising administering a therapeutically effective amount of at least one HER inhibitor to said patient. The HER inhibitor can be e.g. a EGFR/HER2 dual tyrosine kinase inhibitor, PKC412 or imatinib mesylate.

### Summary

The scope of this invention is defined by the claims. Embodiments in the description relating to methods of treatment are not covered by the claims. Any "embodiment" or "example" which is disclosed in the description but is not covered by the claims should be considered as presented for illustrative purpose only.

Described herein, but not forming part of the claimed invention, is a method for treating a patient suffering from chronic inflammatory injury, metaplasia, dysplasia or cancer of an epithelial tissue, which method comprises administering to the patient an anti-PESC agent that selectively kills or inhibits the proliferation or differentiation of pathogenic epithelial stem cells (PESCs) relative to normal epithelial stem cells in the tissue in which the PESC is found. Representative epithelial tissues include pulmonary, genitourinary, gastrointestinal, pancreatic and hepatic tissues.

Also described herein, but not part of the claimed invention, is a method of reducing proliferation, survival, migration, or colony formation ability of PESCs in a subject in need thereof comprising contacting the PESC with a therapeutically effective amount of an anti-PESC agent that selectively kills or inhibits the proliferation or differentiation of a PESC population relative to normal epithelial stem cells in the tissue in which the PESCs are found.

For example, described herein is a method for treating a patient suffering from one or more of esophagitis (including Eosinophilic esophagitis or EoE), Barrett's Esophagus, esophageal dysplasia or esophageal cancer, which method comprises administering to the patient an anti-PESC agent that selectively kills or inhibits the proliferation or differentiation of Barrett's Esophagus stem cells (BESC) relative to normal esophageal stem cells. In certain embodiments, the patient presents with esophagitis. In certain embodiments, the patient presents with Barrett's Esophagus. In certain embodiments, the patient presents with esophageal dysplasia. In certain embodiments, the patient presents with esophageal cancer. In certain embodiments, the patient presents with esophageal carcinoma, such as esophageal adenocarcinoma or esophageal squamous cell carcinoma.

The present invention provides a drug eluting device for use as part of a treatment for one or more of chronic inflammatory injury, metaplasia, dysplasia or cancer of an epithelial tissue, such as for treating one or more of esophagitis, Barrett's esophagus, esophageal dysplasia or esophageal cancer, which device comprises drug release means including ponatinib and an anti-PESC agent selected from the group consisting of delanzomib and JIB-04, which device when deployed in a patient positions the drug release means proximal to the luminal surface of the esophagus and releases the ponatinib and the anti-PESC agent in an amount sufficient to achieve a therapeutically effective exposure of the luminal surface to these agents. In certain embodiments, the patient presents with esophagitis. In certain embodiments, the patient presents with Barrett's Esophagus. In certain embodiments, the patient presents with esophageal dysplasia. In certain embodiments, the patient presents with esophageal cancer. In certain embodiments, the patient presents with esophageal carcinoma, such as esophageal adenocarcinoma or esophageal squamous cell carcinoma. Examples of drug eluting devices are drug eluting stents, drug eluting collars and drug eluting ballons.

In other embodiments, there are provided drug eluting devices that can be implanted proximal to the diseased portion of the luminal surface of the esophagus, such as implanted extraluminally (*i.e.,* submucosally or in or on the circular muscle or longitudinal muscle) rather than intraluminally.

In certain embodiments, the anti-PESC agent is administered during or after endoscopic ablation therapy, such as radiofrequency ablation, photodynamic therapy or cryoablation of esophageal tissue.

The present invention provides a drug eluting device as defined in independent claim 1, which device when deployed in a patient positions the drug release means proximal to target epithelial tissue and releases the ponatinib and the anti-PESC agent selected from the group consisting of delanzomib and JIB-04 in an amount sufficient to achieve a therapeutically effective exposure of the target epithelial tissue to the agents.

In certain embodiments, the target epithelial tissue is esophageal tissue.

In certain embodiments, the target epithelial tissue is an epithelial-derived tumor, such as an ovarian tumor, a lung tumour, a gastric tumor or an esophageal tumor, or a metastatic site thereof.

For instance, the drug eluting device can produce at least a minimally effective concentration (MEC) of the anti-PESC agent in the target epithelial tissue to which it is applied to which it is applied for at least 30 minutes, more preferably at least 60, 120, 180, 240 or even 300 minutes.

For instance, the drug eluting device can produce anti-PESC agent concentration in the esophageal tissue to which it is applied with T1/2 of at least 2 hours, more preferably at least 4, 6, 8, 10 or even 12 hours.

In certain embodiments, the drug eluting device produces a systemic concentration of the anti-PESC agent which is less than 1/3^{rd} the maximum tolerated does (MTD) for that agent, and even more preferably less than 1/5^{th}, 1/10^{th} , 1/20^{th}, 1/50^{th} or even 1/100^{th} the maximum tolerated does (MTD) for that agent.

In certain embodiments, the drug eluting device is for treating one or more of esophagitis, Barrett's esophagus, esophageal dysplasia or esophageal cancer, which device comprises drug release means including an Anti-BESC Agent that selectively kills or inhibits the proliferation or differentiation of Barrett's Esophagus stem cells (BESC) relative to normal esophageal stem cells, which device when deployed in a patient positions the drug release means proximal to the luminal surface of the esophagus and releases the agent in an amount sufficient to achieve a therapeutically effective exposure of the luminal surface to the agent.

Exemplary drug eluting devices include biodegradable stents, self-expandable stents, such as a self-expandable metallic stent (SEMS) or self-expandable plastic stent (SEPS), chips and wafers for submucusal implantation, and the like.

In other embodiments, the drug eluting device is a device for extraluminal placement, such as a microneedle cuff.

In certain embodiments, the anti-PESC agent is co-administered with an analgesic, and an anti-infective or both. These may be administered as separate formulation, or optionally, may be the anti-PESC agent is co-formulated with the analgesic or the anti-infective or both.

In certain embodiments, the anti-PESC agent is delivered by a drug eluting device that is a drug eluting stent.

In certain embodiments, the anti-PESC agent is delivered by a drug eluting device that is a balloon catheter having a surface coating including the agent.

According to the claimed invention, the anti-PESC agent is a selected from the group consisting of delanzomib and JIB-04. The remaining anti-PESC agents referred to herein are provided for reference purposes.

| | |
|---|---|
| 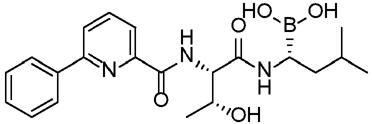 Delanzomib (CEP-18770); | 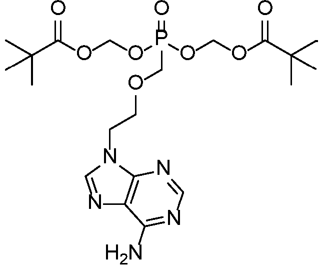 Adefovir Dipivoxyl; |
| 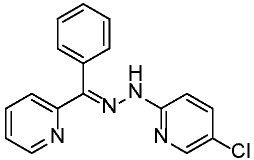 JIB04; | 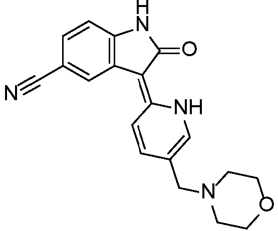 AZD1080. |
| 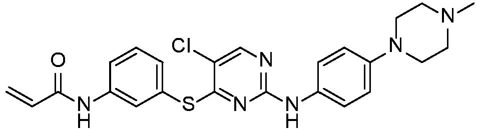 WZ8040; | 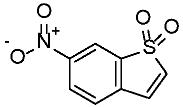 Stattic; |
| 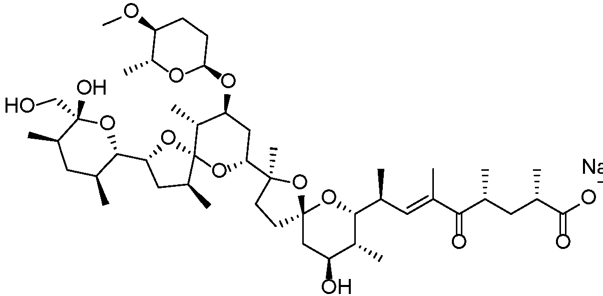 Nanchangmycin; and | |

The anti-PESC agent is administered with an ESO Regenerative agent. The ESO Regenerative agent is pan-inhibitor of ABL kinase inhibitor, and is
In certain embodiments, the anti-PESC agent and the ESO Regenerative agent are administered to the patient as separate formulations.

In certain embodiments, the anti-PESC agent and the ESO Regenerative agent are co-formulated together.

In certain embodiments, the methods, preparations and devices described herein are intended (and appropriate) for use in human patients.

### Description of the Figures

**Figures 1A-G**. Establishment of HTS screening on BE stem cells.
   **Figure 1A****.** Schematic of chemical screening pipeline. 1 mm biopsy of BE lesion was processed and mixed BE stem cells (Krt7+) and ESO stem cell (Krt5+) were cloned and culture in StemECHO cell culture system. Single cell derived pedigrees of Krt5+ ESO stem cells and Krt7+ BE stem cells were established, labeled with GFP and subject to chemical screening. Drugs that eradicate the BE stem cells were identified as hits.
   **Figure 1B****.** Summary of the chemical libraries being used for the screenings.
   **Figure 1C****.** Sensitivity clustering of cell lines used in the screening. BE stem cells and ESO stem cells showed overall distinct sensitivity to chemicals included in the screenings.
   **Figure 1D****.** Left, Scatter plot comparing median survival rate of BE stem cells and ESO stem cells. Right, Ponatinib promoted the proliferation of ESO stem cells.
   **Figure 1E****.** Left, Representative images of ESO and BE stem cells in the absence or presence of Ponatinib. Right, Dosage response of BE and ESO stem cells towards Ponatinib.
   **Figure 1F****.** Representative image of a scanned 384 plate showing the effects of the chemicals on GFP labeled stem cells.
   **Figure 1G****.** Example of converting green binary overlay to the heatmap representing the total area of colonies in each individual well.
**Figures 2A-F**. Synergistic HTS screening to identify dual-function drug combinations.

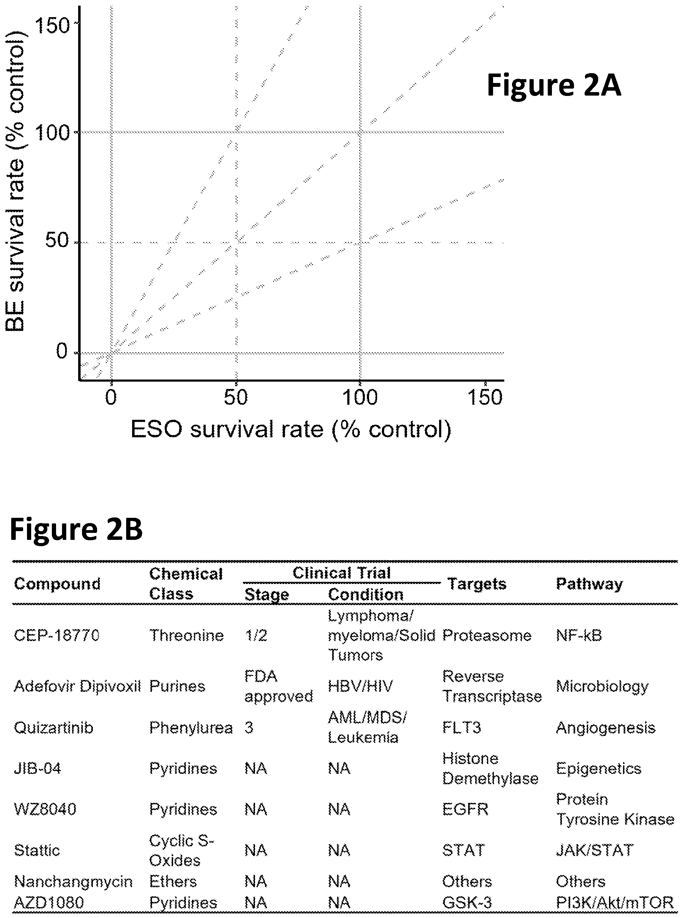

   **Figure 2A****.** Scatter plot display of the HTS result of synergistic HTS. Selected eight top hits were highlighted in red.
   **Figure 2B****.** Summary of the information of selected top hits.

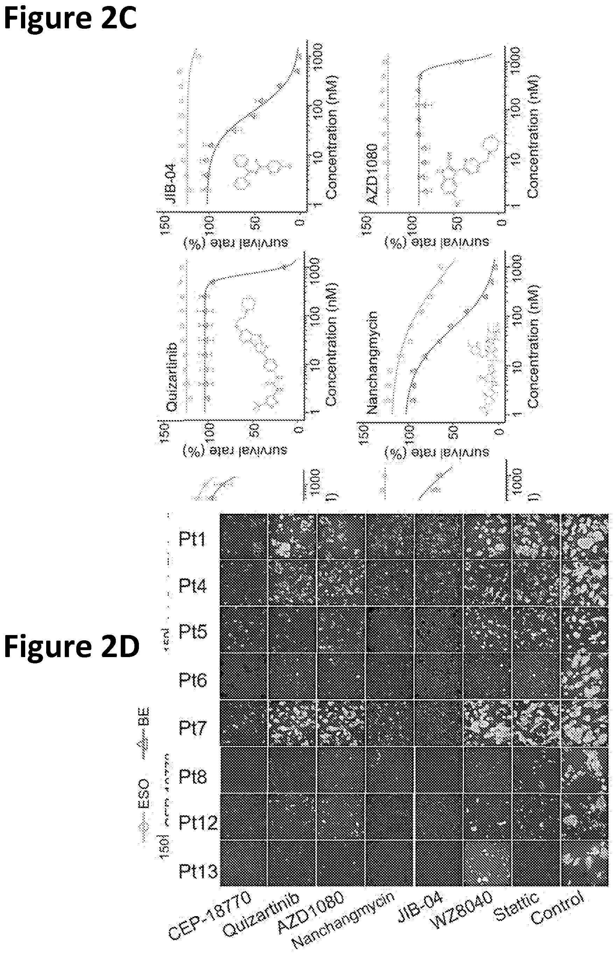

   **Figure 2C****.** Dosage response curves of the top eights hits validated their differential effects to BE and ESO stem cells.

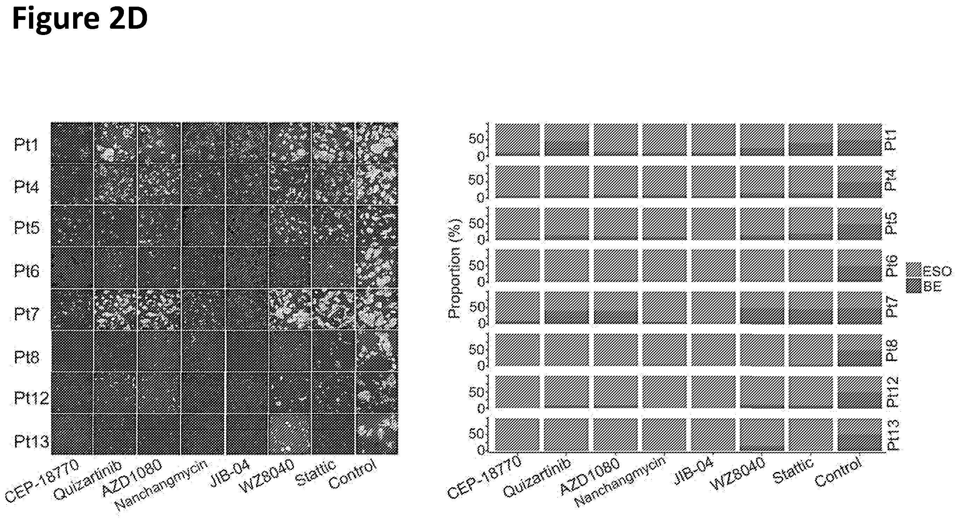

   **Figure 2D****.** *Left,* Scanned images of BE (Krt7+, Green)/ESO (Krt5+, Red) co-culture system for the validation of seven selected drugs on eight patients. *Right,* Quantitative analysis of the scanned images.

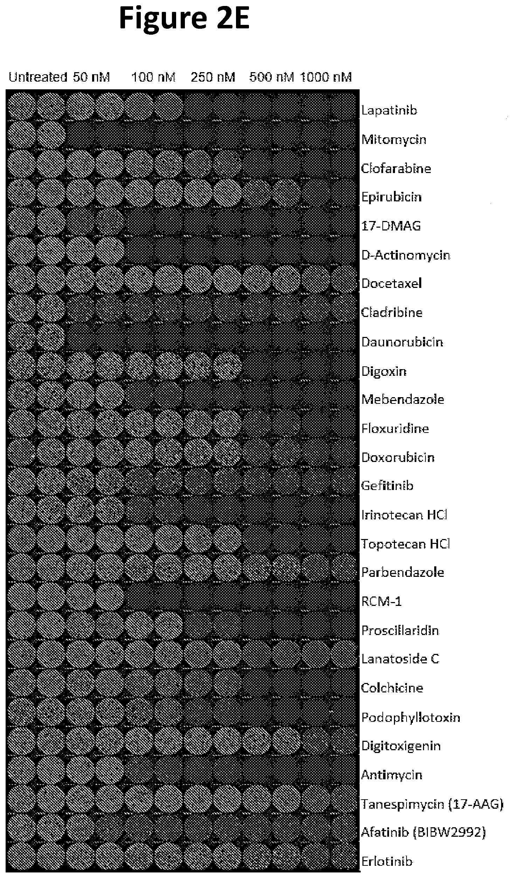

   **Figure 2E****.** Scanned image of GFP labeled BE stem cells treated with a gradient of selected hits from HTS.

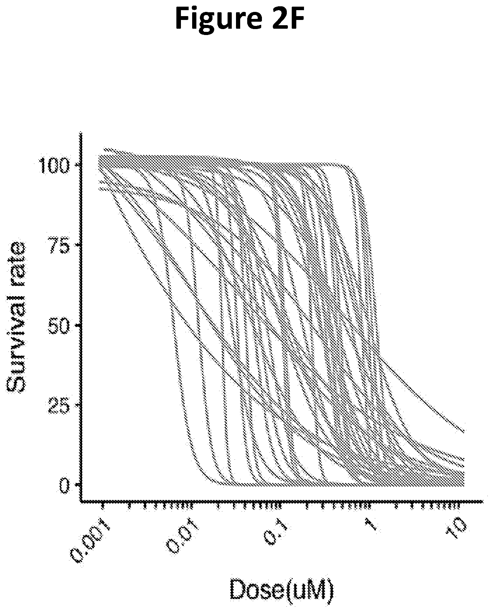

   **Figure 2F****.** Dosage curves of the hits showed that all selected ones were validated to eradicate BE stem cells. Numeric values obtained from GFP signal threshold image analysis were used to generate dose response curves.

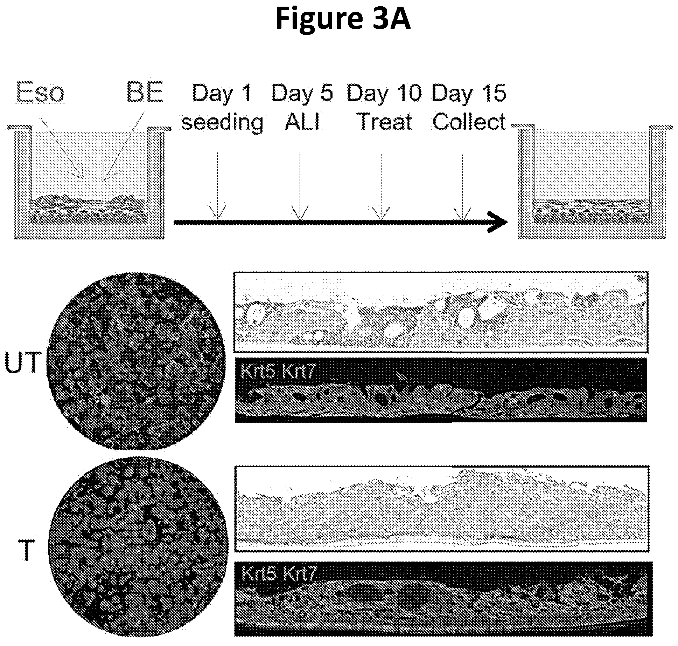

**Figures 3A-E**. Validation of selected hits in 3D and mouse models.

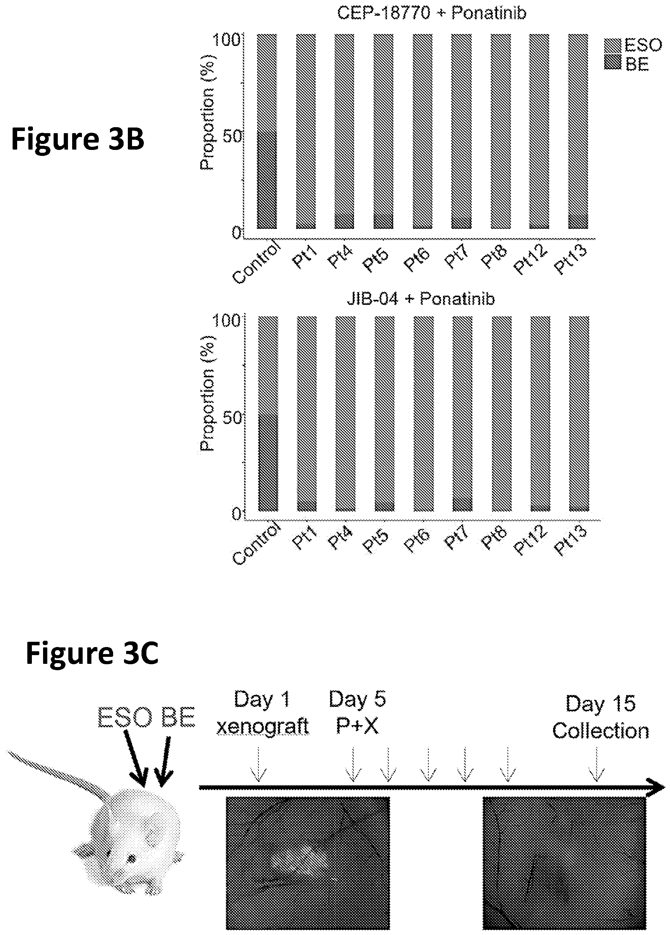

   **Figure 3A****.** Upper, Schematic of the 3D culture validation. Lower, Representative images of top view and histological sections of untreated and treated 3D co-culture structures. BE stem cells (Krt7+, Green) and ESO stem cells (Krt5+, Red) co-existed in the absence of selected drug combination. Following the treatment, BE stem cells (Green) were eradicated while ESO stem cells (Red) compensated the blanked area. **Figure 3B****.** Quantitative analysis of 3D co-culture treatment images of top view from eight patients.
   **Figure 3C****.** Schematic of drug combination validation in a mouse model. Mice were co-injected subcutaneously with a mixture of BE (GFP labeled) and ESO stem cells. Following the serial treatments, the GFP+ BE stem cells became invisible.

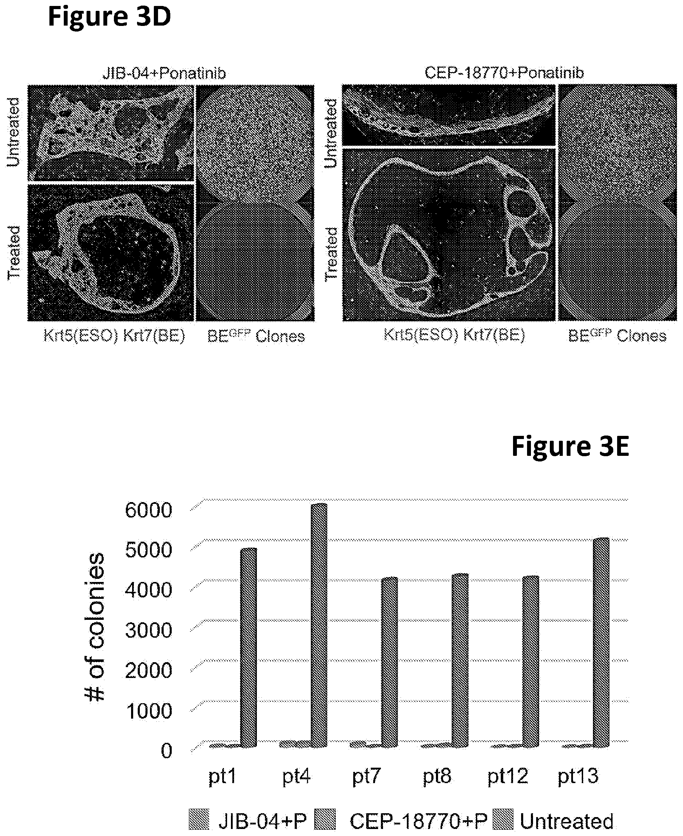

   **Figure 3D****.** Histological analysis of BE stem cells (Krt7+, Green)/ESO stem cells (Krt5+, Red) co-transplanted structures from treated and non-treated mice.
   **Figure 3E****.** Quantitative analysis of clonogenic assay performed by processing the untreated and treated transplants and culturing in StemECHO culture system.

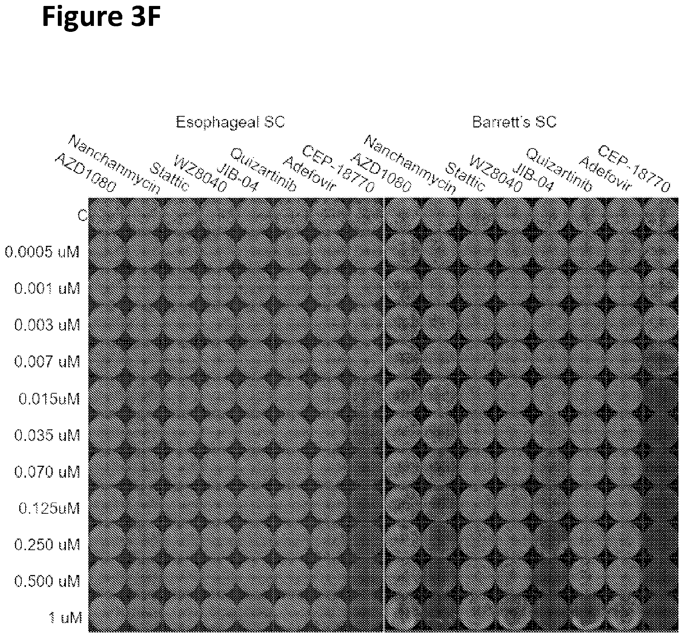

   **Figure 3F****.** Scanned images of BE stem cells and ESO stem cells treated with a range of dosages of eight selected hits together with 1 µM Ponatinib. Their differential effects on BE and ESO stem cells were validated except AZD1080 that appeared to be the false positive hit in the screening.

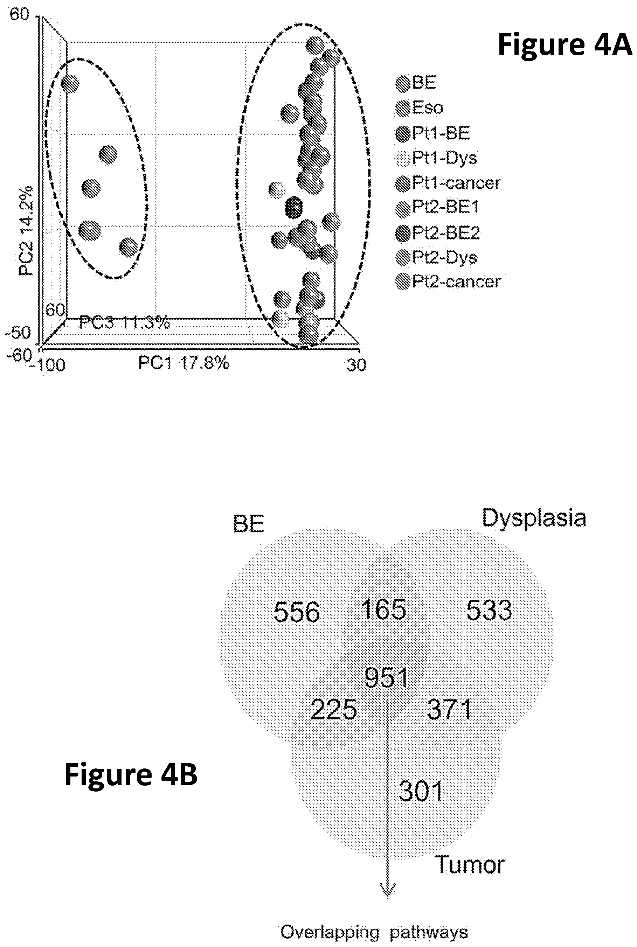

**Figures 4A-E**. Drug combinations eradicated patient-matched BE, dysplasia and cancer.
   **Figure 4A****.** PCA map of stem cell gene expression from Barrett's, dysplastic, tumor and normal esophagus.
   **Figure 4B****.** Venn diagrams of overlapped genes among BE, dysplasia and cancer stem cells versus ESO stem cells.

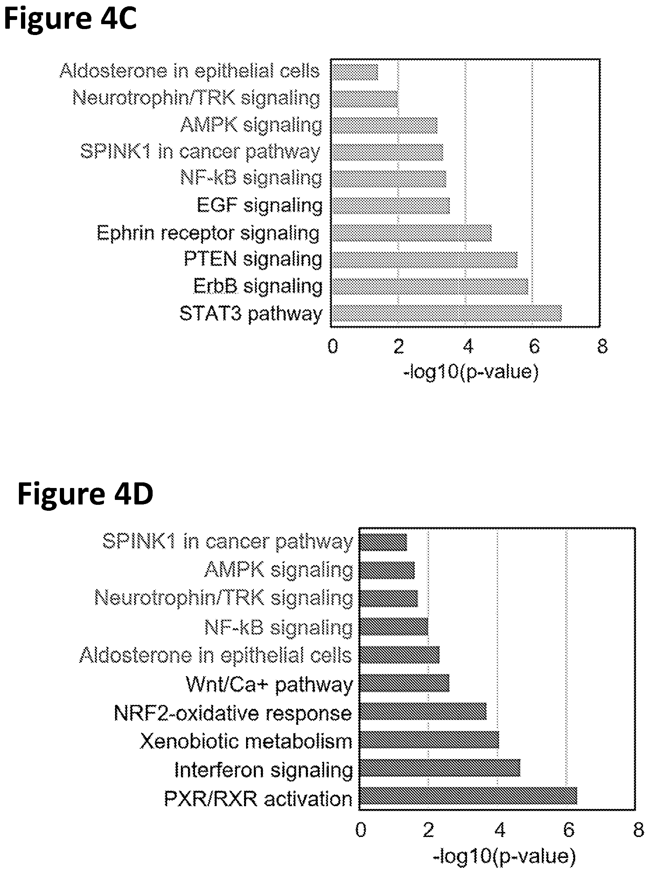

   **Figure 4C****.** Common pathways enriched in BE, dysplasia and cancer.
   **Figure 4D****.** Pathways targeted by the hits identified in HTS.

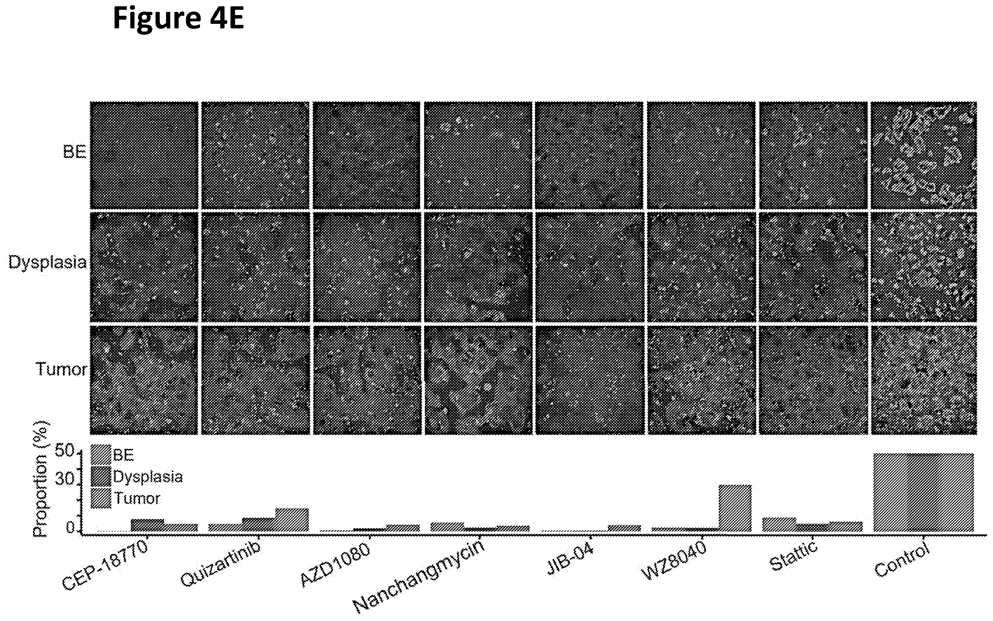

   **Figure 4E****.** Upper, Scanning images of co-cultured BE stem cells (Krt7+, Green) and ESO stem cells (Krt5+, Red) treated with seven different drug combinations. Lower, Quantitative analysis of the scanned images showed that patient-matched BE, dysplasia and cancer stem cells could be eradicated with the same drug combinations.

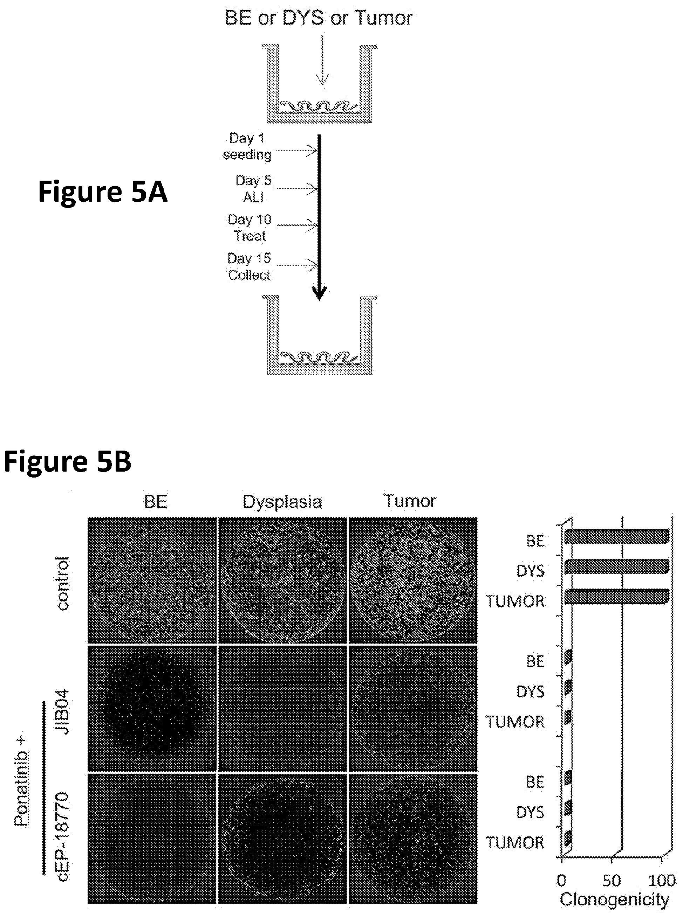

**Figures 5A-G**. CEP-18770 and JIB04 together with Ponatinib eradicated BE, dysplasia and cancer stem cells *in vitro* and *in vivo.*
   **Figure 5A****.** Schematic of 3D culture validation on patient-matched BE, dysplasia and cancer stem cells.
   **Figure 5B****.** Left, Scanned images of top view of BE, dysplasia and cancer stem cells (green) generated ALI structures in the presence of absence of drug combinations. Right, Quantitative analysis of the images of ALI structures showed that CEP-18770 and JIB04 combined with Ponatinib eradicated BE, dysplasia and cancer stem cells.

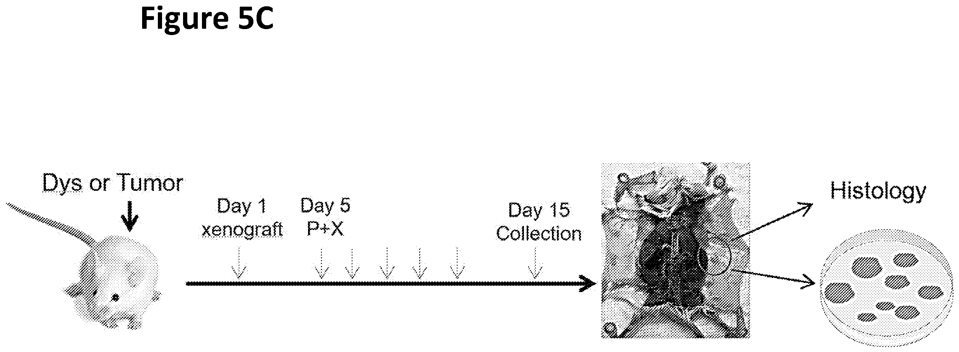

   **Figure 5C****.** Schematic of *in vivo* validation of drug combinations in mice xenografted with dysplasia and cancer stem cells. Following the treatment, the xenografts were processed for histological analysis or clonogenic assays to detect the existence of stem cells.

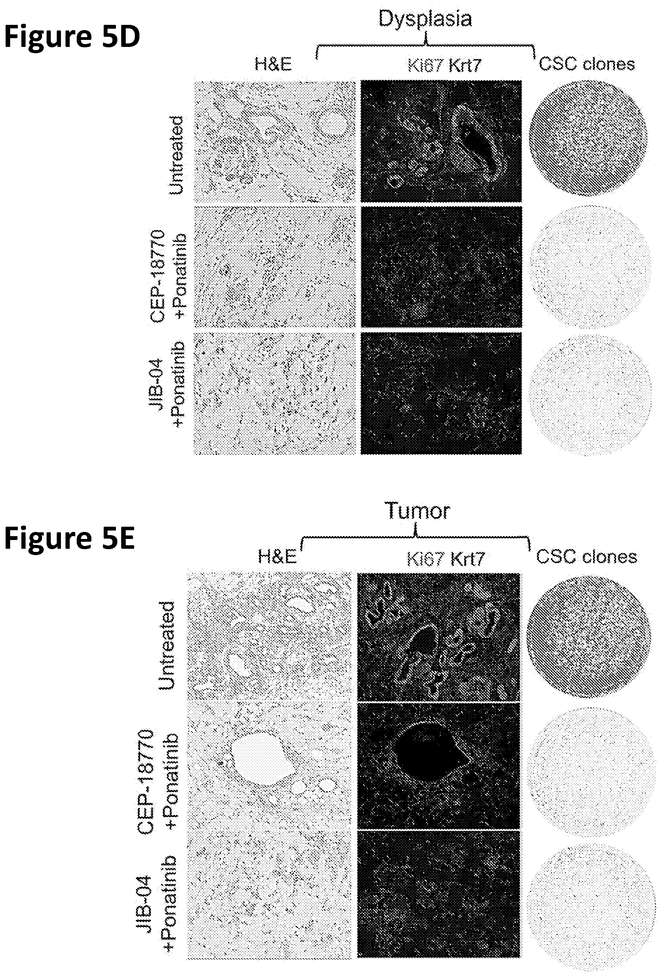

   **Figure 5D****.** Dysplastic structures (Krt7+, Green/Ki67+, Red) were diminished following CEP-19770/Ponatinib or JIB04/Ponatinib treatment. Consistently, stem cells were not cloned from treated structures as shown in scanned rhodamine staining.
   **Figure 5E****.** Cancer structures (Krt7+, Ki67+) were diminished following the drug treatment. Rhodamine staining showed the cancer stem cells were not cloned from the treated structures.

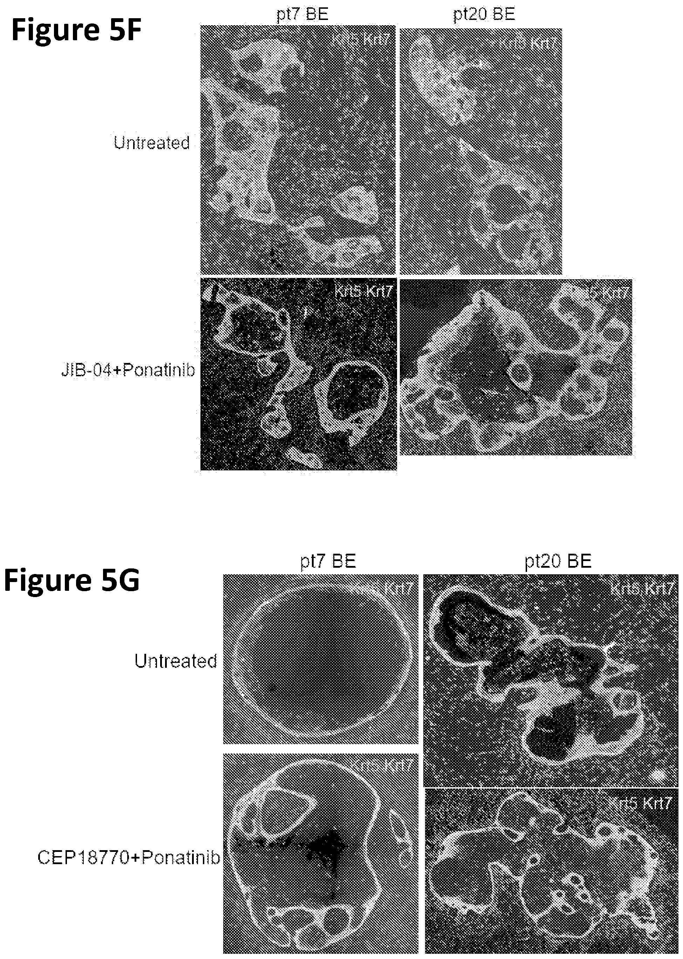

   **Figures 5F and 5G****.** BE stem cells (Krt7+, Green) and ESO stem cells (Krt5+, Red) were co-injected into the NSG mice and treated with JIB04 or CEP-18770 with Ponatinib. The tissues were collected and fixed for histological analysis. BE stem cells were not detected in the treated structures while the Krt5+ ESO structures were visibly more robust in the treated samples.

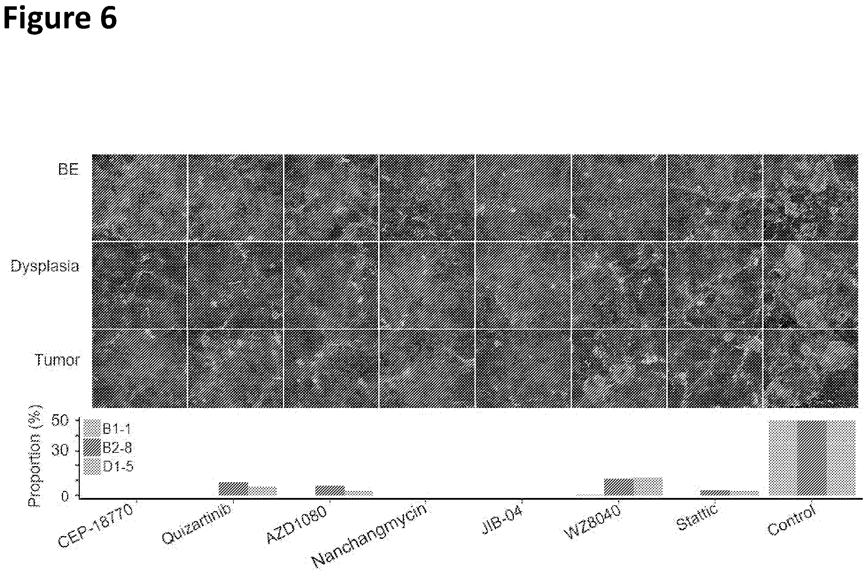

**Figure 6****.** Patient-matched BE, dysplasia and cancer stem cells (Krt7+, Green) were mixed with Krt5+ (red) ESO cells and co-cultured for five days and then treated with seven selected drugs together with 1 µM Ponatinib. Scanning image and quantitative analysis showed that all seven drug combinations reduced the growth of BE, dysplasia and cancer stem cell growth, while JIB04 and CEP-18770 remained the most effective drugs.

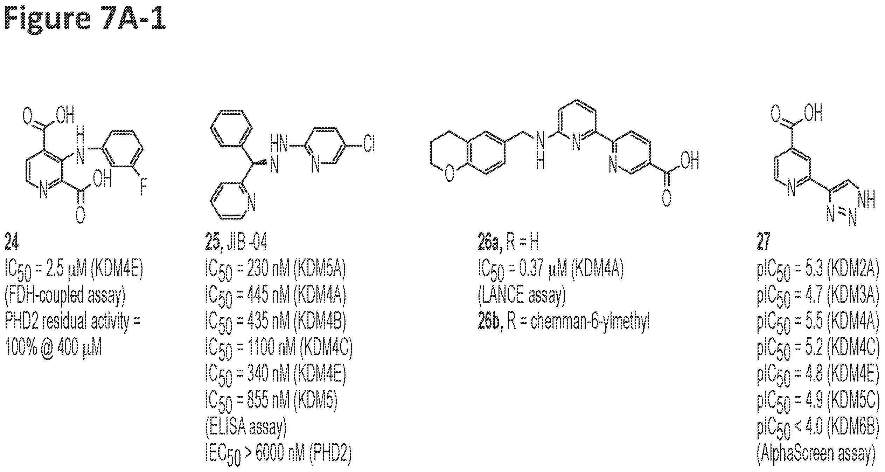

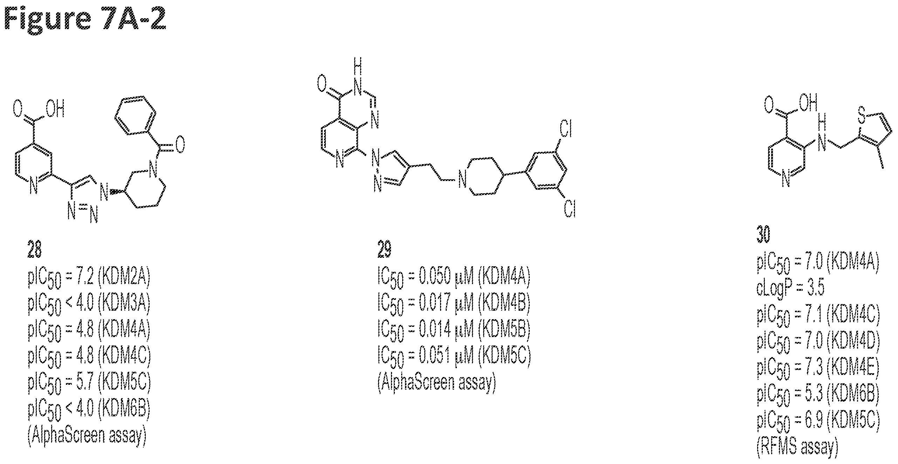

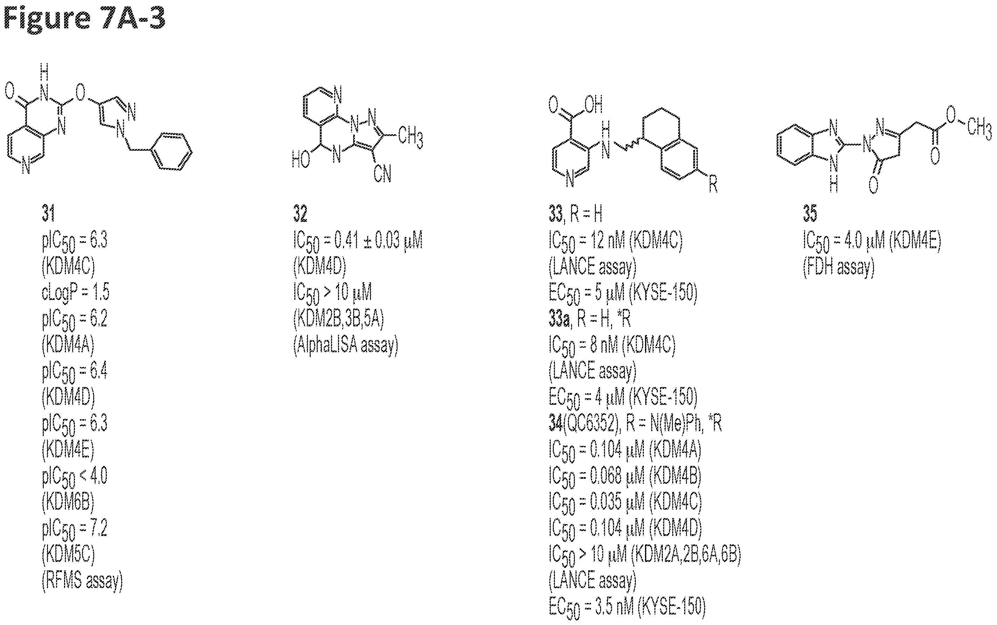

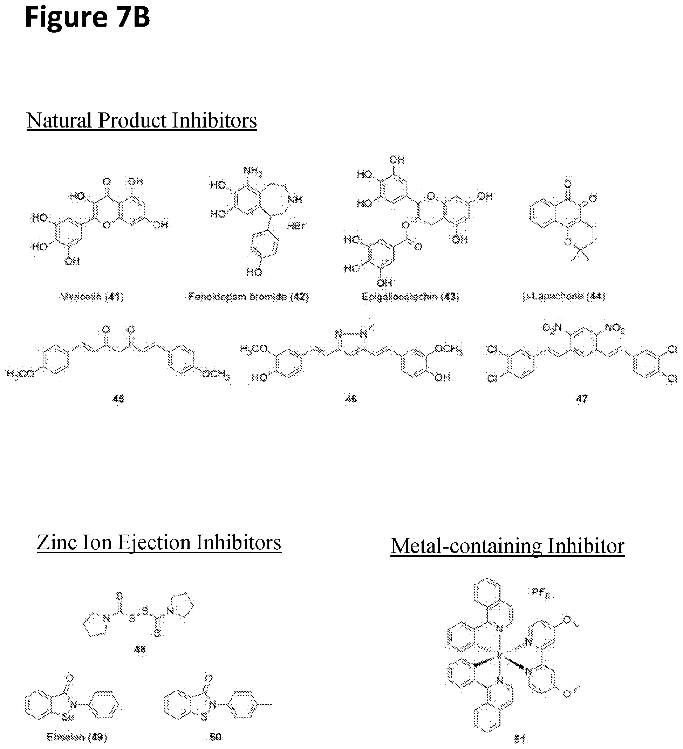

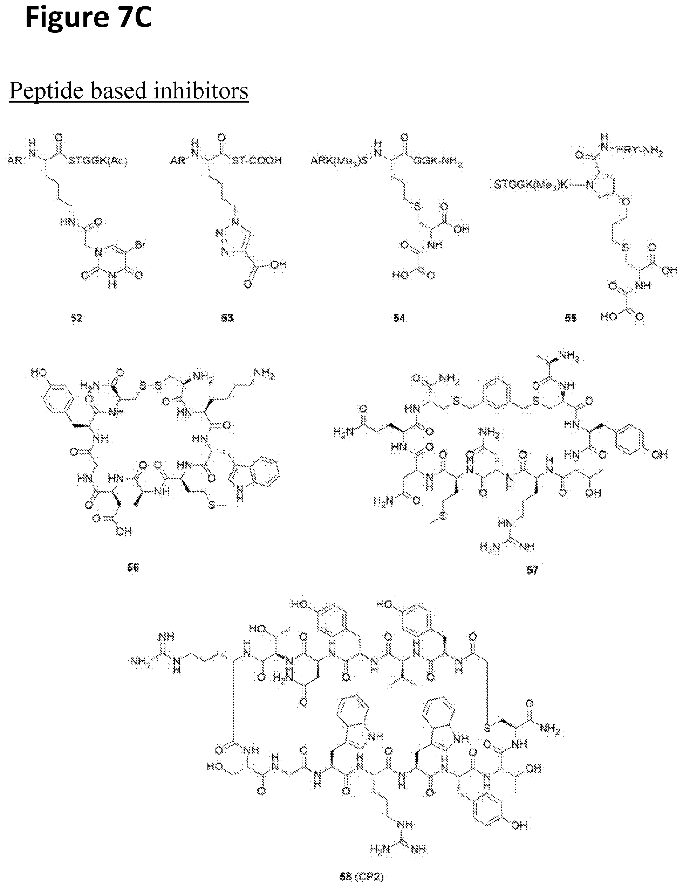

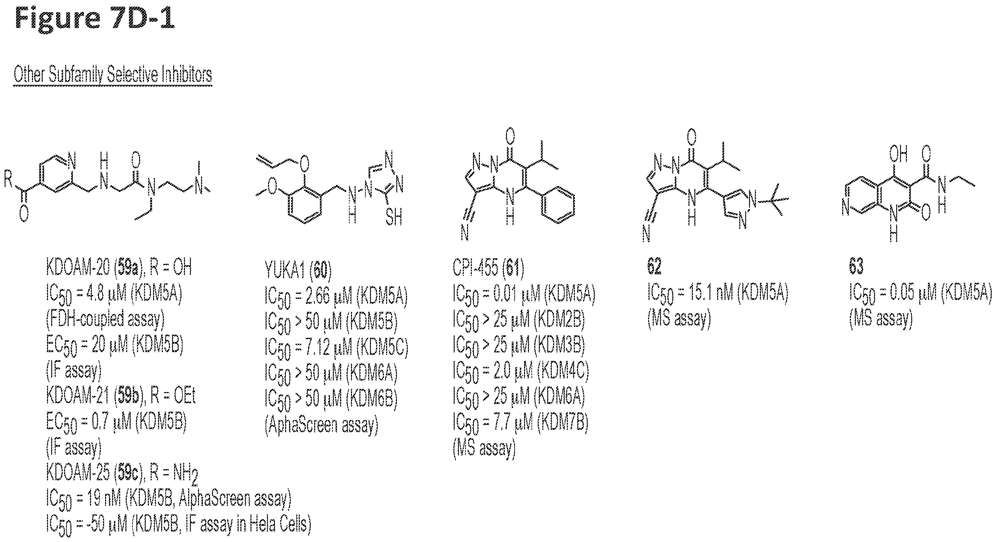

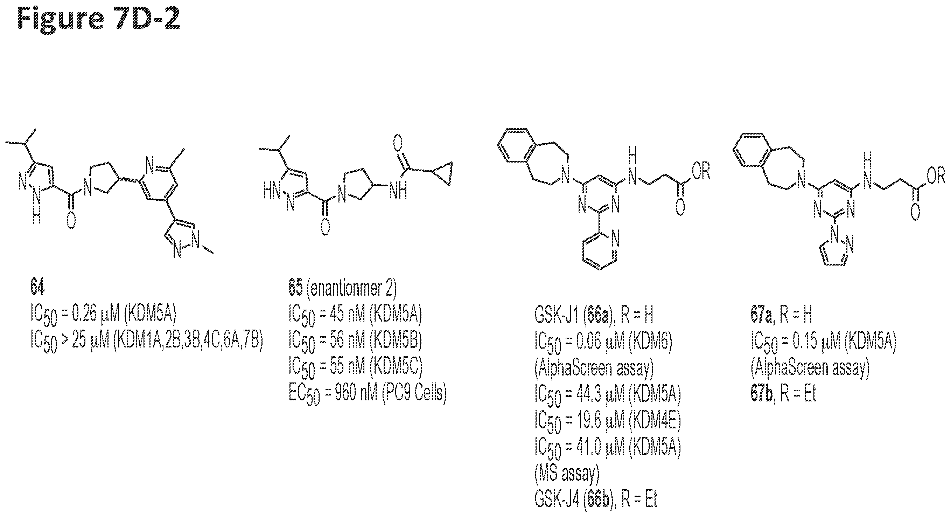

**Figures 7A-7D**. Exemplary classes and illustrative structures of histone deacetylase inhibitors useful in the present disclosure.
**Figures 8****.** Is a diagram representing the continuum in certain epithelial tissues of metaplasia to dysplasia to cancer.
**Figures 9****.** Is a diagram showing the statistically increasing risk of a patient developing esophageal adenocarcinoma as disease progresses from Barrett's esophagus to high grade dysplasia.

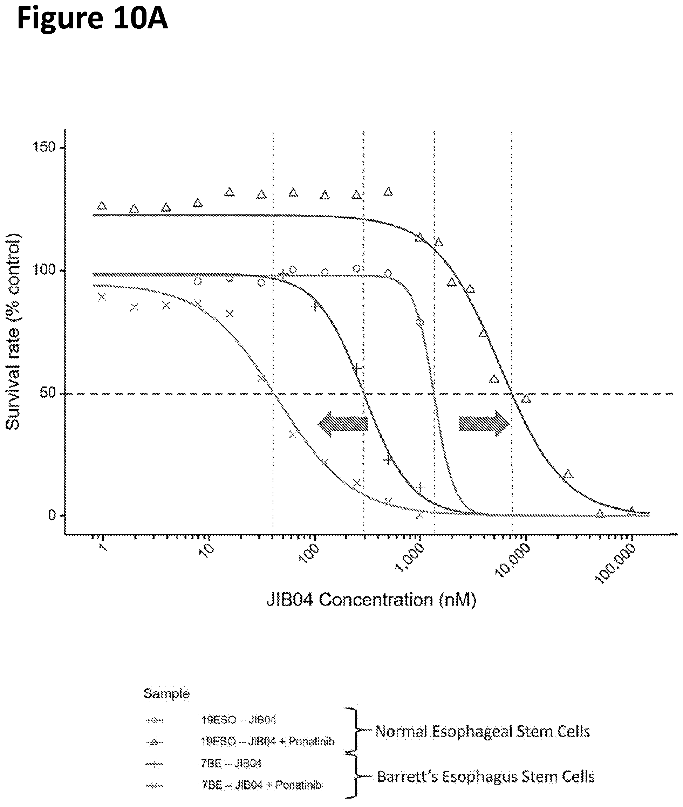

**Figures 10A****.** Demonstrates the synergy in the combination of JIB04 with ponatinib in killing pathogenic BE stem cells relative to normal esophageal stem cells. Projected therapeutic index is greater than 200 fold.

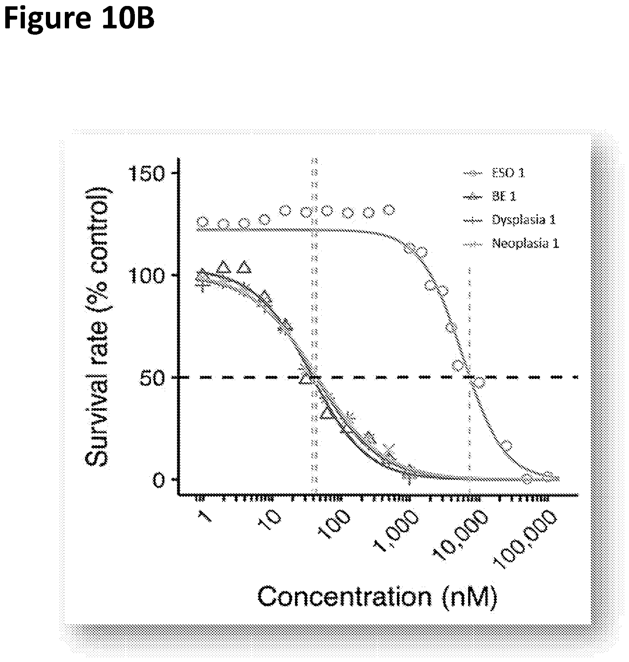

**Figures 10B****.** Demonstrates the synergy in the combination of JIB04 with ponatinib in killing both BE stem cells, as well as stem cells from biopsies graded as dysplasia and Esophageal Adenocarcinoma (EAC). Projected therapeutic index is greater than 100 fold.

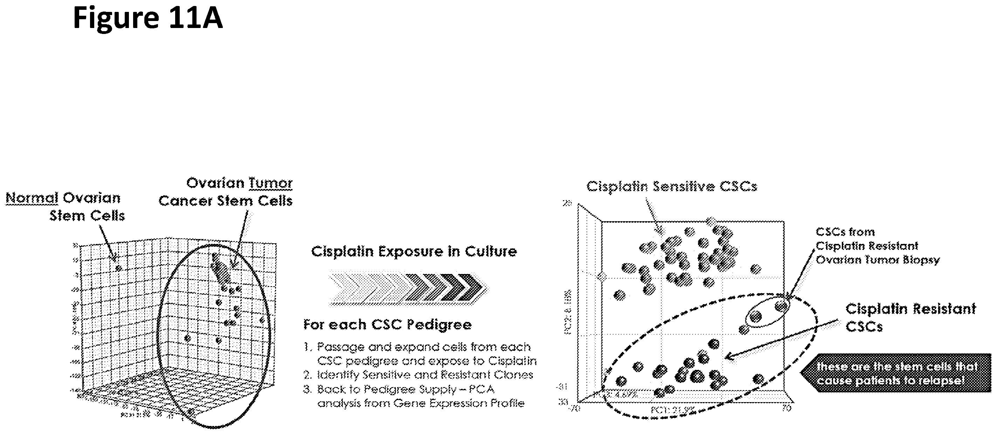

**Figures 11A****.** Pathogenic stem cells isolated from high grade ovarian tumor biopsies obtained from drug naive patients already include cisplatin-resistant stem cell populations. PCA analysis of cisplatin-resistant stem cell populations from drug naive patients indicates that these cells cluster with pathogenic stem cells from stem cells isolated from cisplatin-resistant tumors from patients having relapsed or failed cisplatin therapy.

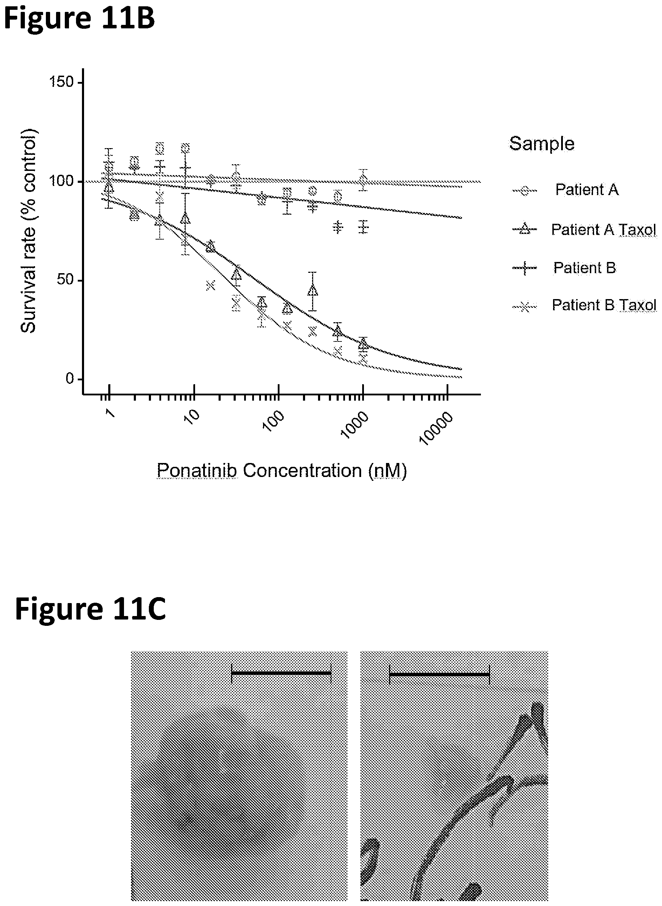

**Figures 11B****.** Demonstrates the synergy in the combination of taxol with ponatinib in killing taxol-resistant ovarian stem cells *in vitro.*
**Figures 11C****.** Demonstrates the synergy in the combination of taxol with ponatinib in reducing tumor volume taxol-resistant ovarian stem cells in xenograft animal models.

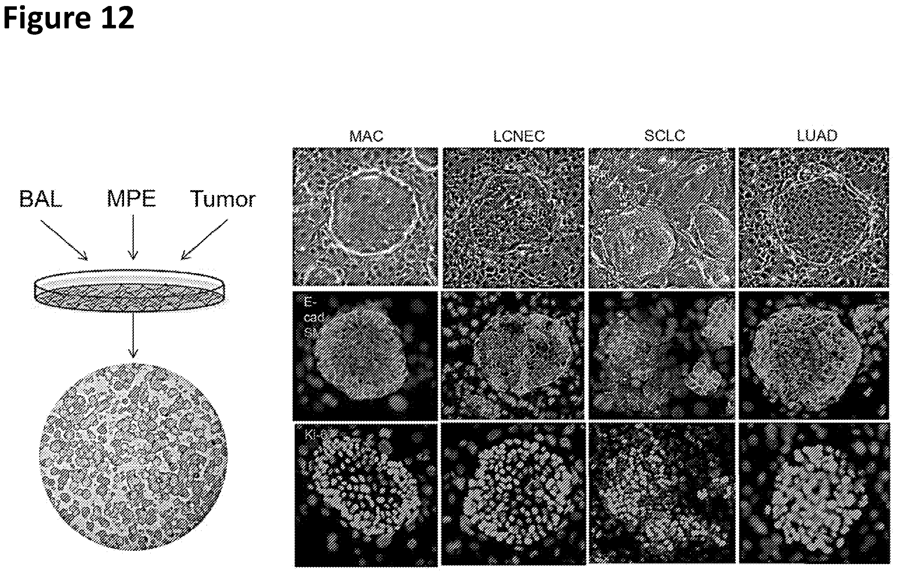

**Figures 12****.** Pathogenic stem cells can be cloned and cultured from lung cancers (both small cell and non-small cell lung cancer). These pathogenic stem cells, along with normal regenerative lung stem cells, can be adapted to high throughput screening plates.

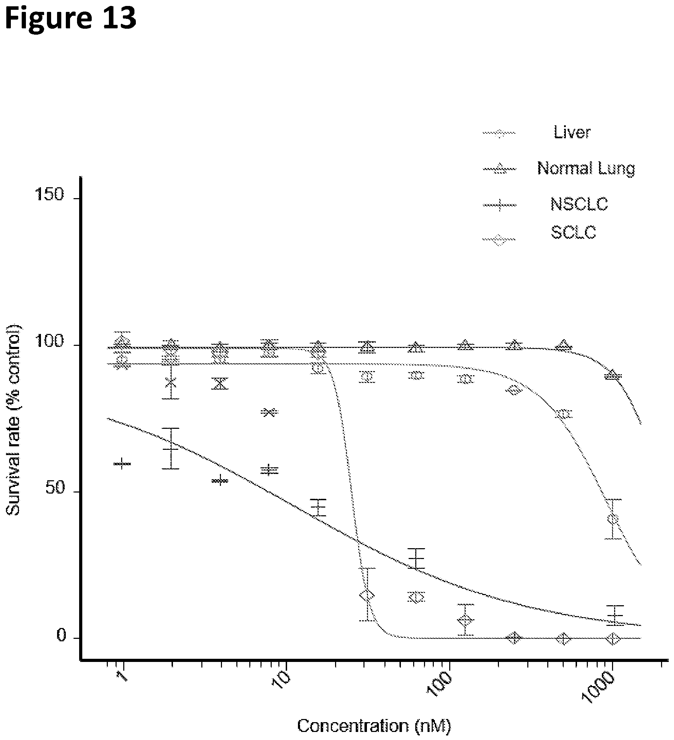

**Figures 13****.** Demonstrates the synergy in the combination of JIB04 with ponatinib in killing both NSCLC and SCLC stem cells relative to normal lung epithelial stem cells.

### Description of Certain Embodiments

### I. Overview

Barrett's Esophagus holds a pivotal position at the interface of cancer biology and patient care. Barrett's was first discovered in 1950's and associated with risk for adenocarcinoma in the 1970's. Barrett's has become a paradigm for precancerous lesions giving rise to progressively more advanced lesions in a process requiring many years supporting an overall escalation model whereby non-cancerous lesions undergo long-term processes of stochastic changes some of which yield more sinister and determinant transitions to low- and high-grade dysplasia which then rapidly and almost inexorably evolve to malignant disease. The recognition of the importance of preemptive therapies that target these premalignant lesions is the foundation of cancer prevention. If true, the clinical solution to preventing the onset of esophageal adenocarcinoma would be simple and direct: ablate Barrett's before it can evolve to more aggressive lesions.

The advance of the development of targeted therapies for Barrett's requires conceptual advance of the origin of Barrett's and the recognition of the existence of Barrett's stem cells. If the premalignant stages of EAC represent the only tractable solution to this disease, it is essential to solve the mystery of the origin of BE and develop new therapeutic strategies specifically targeting its stem cells. However, the ontogeny of BE has been an intriguing puzzle with various hypotheses involving transcommitment of esophageal squamous stem cells, migration from lower gastrointestinal sites, the reparative emergence of submucosal glands, dissemination from bone marrow. We recently showed that BE originated from the opportunistic growth of residual embryonic cells pre-existing at gastroesophageal junction (Wang et al, Cell. 2011 Jun 24;145(7):1023-1035). In addition, using the ground state stem cell technology that enabled us to clone stem cells of the normal human gastrointestinal tract, we demonstrated the existence of the stem cells in BE (Yamamoto et al., Nat Commun. 2016 Jan 19;7:10380) and suggested they are the key elements to target in a therapeutic program designed to prevent the development and progression of this irreversible and dangerous metaplasia.

In order to uncover drugs specifically targeting BE stem cells that might synergize with physical ablation protocols to further reduce recurrent disease, provided herein is a multiplexed screening of established and experimental drugs or combinations thereof to identify compounds and combinations of compounds that selectively target the particular pathways that dominate the survival of these BE lesions. These BE stem cells were used in hybrid models with normal epithelial squamous stem cells to model the potential ability of such drug combinations to alter the competitive status of such lesions in the distal esophagus.

Also provided herein are screening methods that show the similar selective vulnerabilities of the stem cells of patient-matched BE, dysplasia and EAC, which suggest the broad usage of the pharmacological compositions that would augment physical ablation or mucosal dissection therapies. Indeed, as demonstrated by the data presented herein, the differential sensitivity of the pathogenic stem cells to single agents or combination therapies is carried across multiple tissues and across metaplasia, dysplasia or tumor samples from those tissues.

### II. Definitions

Unless otherwise stated, the following terms used in the specification and claims are defined for the purposes of this Application and have the following meaning:
"Alkyl" means a linear saturated monovalent hydrocarbon radical of one to six carbon atoms or a branched saturated monovalent hydrocarbon radical of three to six carbon atoms, *e.g.,* methyl, ethyl, propyl, 2-propyl, butyl (including all isomeric forms), pentyl (including all isomeric forms), and the like. The term "alkyl," as used herein, in synonymous with the term "aliphatic."

The term "alkenyl" as used herein describes groups which are preferably lower alkenyl containing from two to eight carbon atoms in the principal chain and up to 20 carbon atoms. They may be straight or branched chain or cyclic and include ethenyl, propenyl, isopropenyl, butenyl, isobutenyl, hexenyl, and the like.

The term "alkynyl" as used herein describes groups which are preferably lower alkynyl containing from two to eight carbon atoms in the principal chain and up to 20 carbon atoms. They may be straight or branched chain and include ethynyl, propynyl, butynyl, isobutynyl, hexynyl, and the like.

"Alkylsulfonyl" means a -SO₂R radical where R is alkyl as defined above, *e.g.,* methylsulfonyl, ethylsulfonyl, and the like.

"Alkoxy" means an -OR radical where R is alkyl as defined above, *e.g.,* methoxy, ethoxy, propoxy, or 2-propoxy, n-, iso-, or tert-butoxy, and the like.

"Aminoalkyl" means a linear monovalent hydrocarbon radical of one to six carbon atoms or a branched monovalent hydrocarbon radical of three to six carbons substituted with at least one, preferably one or two, -NRR where R is hydrogen, alkyl, or -COR^{a} where R^{a} is alkyl, each as defined above, and R is selected from hydrogen, alkyl, hydroxyalkyl, alkoxyalkyl, aryl, aralkyl, heteroaryl, heteroaralkyl, or haloalkyl, each as defined herein, *e.g.,* aminomethyl, methylaminoethyl, 2-ethylamino-2-methylethyl, 1,3-diaminopropyl, dimethylaminomethyl, diethylaminoethyl, acetylaminopropyl, and the like.

"Aminosulfonyl" means a -SO₂NRR' radical where R is independently hydrogen, alkyl, hydroxyalkyl, alkoxyalkyl, or aminoalkyl, each as defined herein and R' is hydrogen, alkyl, cycloalkyl, cycloalkylalkyl, aryl, aralkyl, heteroaryl, heteroaralkyl, heterocyclyl, heterocyclylalkyl, hydroxyalkyl, alkoxyalkyl, or aminoalkyl, each as defined herein, *e.g., -* SO₂NH₂, methylaminosulfonyl, 2-dimethylaminosulfonyl, and the like.

"Acyl" means a -COR radical where R is alkyl, haloalkyl, cycloalkyl, cycloalkylalkyl, aryl, aralkyl, heteroaryl, heteroaralkyl, heterocyclyl, or heterocyclylalkyl, each as defined herein, *e.g.,* acetyl, propionyl, benzoyl, pyridinylcarbonyl, and the like. When R is alkyl, the radical is also referred to herein as alkylcarbonyl.

"Acylamino" means an -NHCOR radical where R is alkyl, haloalkyl, cycloalkyl, cycloalkylalkyl, aryl, aralkyl, heteroaryl, heteroaralkyl, heterocyclyl, or heterocyclylalkyl, each as defined herein, *e.g.,* acetylamino, propionylamino, and the like.

"Aryl" means a monovalent monocyclic or bicyclic aromatic hydrocarbon radical of 6 to 10 ring atoms *e.g.,* phenyl or naphthyl. As used herein, "aryl" and "aromatic" may be used interchangeably.

"Bridged heterocyclyl" means a saturated or unsaturated monovalent bicyclic group of 5 to 10 ring atoms in which one or two ring atoms are heteroatom selected from N, O, or S(0)n where n is an integer from 0 to 2, the remaining ring atoms being C, where some of the rings are created by one or more bridges.

"Cycloalkyl" means a cyclic, saturated, monovalent hydrocarbon radical of three to ten carbon atoms, *e.g.,* cyclopropyl, cyclobutyl, cyclopentyl, or cyclohexyl, and the like. The cycloalkyl ring can optionally be fused to phenyl or monocyclic heteroaryl ring as defined herein. When the cycloalkyl ring is referred to herein as "fused cycloalkyl", it means that the cycloalkyl ring is fused to phenyl or monocyclic heteroaryl ring. When the cycloalkyl ring is referred to herein as "monocyclic cycloalkyl", it means that the cycloalkyl ring is not fused to phenyl or monocyclic heteroaryl ring. As used herein, "cycloalkyl," "carbocycle," and "carbocylyl" may be used interchangeably.

"Carboxy" means -COOH.

"Disubstituted amino" means an -NRR' radical where R and R' are independently alkyl, cycloalkyl, cycloalkylalkyl, acyl, sulfonyl, aryl, aralkyl, heteroaryl, heteroaralkyl, heterocyclyl, heterocyclylalkyl, hydroxyalkyl, alkoxyalkyl, or aminoalkyl, each as defined herein, *e.g.,* dimethylamino, phenylmethylamino, and the like. When R and R' are alkyl, the group is referred to herein as dialkylamino

"Halo" means fluoro, chloro, bromo, or iodo, preferably fluoro or chloro. "Haloalkyl" means alkyl radical as defined above, which is substituted with one or more halogen atoms, preferably one to five halogen atoms, preferably fluorine or chlorine, including those substituted with different halogens, *e.g.,* -CH₂C1, -CF₃, -CHF₂, -CH₂CF₃, - CF₂CF₃, -CF(CH₃)₂, and the like. When the alkyl is substituted with only fluoro, it is referred to in this Application as fluoroalkyl.

"Hydroxyalkyl" means a linear monovalent hydrocarbon radical of one to six carbon atoms or a branched monovalent hydrocarbon radical of three to six carbons substituted with one or two hydroxy groups, provided that if two hydroxy groups are present they are not both on the same carbon atom. Representative examples include, but are not limited to, hydroxymethyl, 2-hydroxy ethyl, 2-hydroxypropyl, 3-hydroxypropyl, I-(hydroxymethyl)-2- methylpropyl, 2-hydroxybutyl, 3-hydroxybutyl, 4-hydroxybutyl, 2,3-dihydroxypropyl, 1- (hydroxymethyl)-2-hydroxyethyl, 2,3-dihydroxybutyl, 3,4-dihydroxybutyl and 2- (hydroxymethyl)-3-hydroxypropyl, preferably 2-hydroxyethyl, 2,3-dihydroxypropyl, or 1- (hydroxymethyl)-2-hydroxyethyl.

"Heterocyclyl" means a saturated or unsaturated monovalent monocyclic group of 4 to 8 ring atoms in which one or two ring atoms are heteroatom selected from N, O, or S(0)ₙ, where n is an integer from 0 to 2, the remaining ring atoms being C. The heterocyclyl ring is optionally fused to a (one) aryl or heteroaryl ring as defined herein provided the aryl and heteroaryl rings are monocyclic. The heterocyclyl ring fused to monocyclic aryl or heteroaryl ring is also referred to in this Application as "bicyclic heterocyclyl" ring. Additionally, one or two ring carbon atoms in the heterocyclyl ring can optionally be replaced by a -CO- group. More specifically the term heterocyclyl includes, but is not limited to, pyrrolidino, piperidino, homopiperidino, 2-oxopyrrolidinyl, 2-oxopiperidinyl, morpholino, piperazino, tetrahydropyranyl, thiomorpholino, tetrahydroisoquinolinyl, and the like. When the heterocyclyl ring is unsaturated it can contain one or two ring double bonds provided that the ring is not aromatic. When the heterocyclyl group contains at least one nitrogen atom, it is also referred to herein as heterocycloamino and is a subset of the heterocyclyl group. When the heterocyclyl group is a saturated ring and is not fused to aryl or heteroaryl ring as stated above, it is also referred to herein as saturated monocyclic heterocyclyl.

"Heteroaryl" means a monovalent monocyclic or bicyclic aromatic radical of 5 to 10 ring atoms where one or more, preferably one, two, or three, ring atoms are heteroatom selected from N, O, or S, the remaining ring atoms being carbon. Representative examples include, but are not limited to, pyrrolyl, thienyl, thiazolyl, imidazolyl, furanyl, indolyl, isoindolyl, oxazolyl, isoxazolyl, benzothiazolyl, benzoxazolyl, quinolinyl, isoquinolinyl, pyridinyl, pyrimidinyl, pyrazinyl, pyridazinyl, triazolyl, tetrazolyl, and the like.

"Monosubstituted amino" means an -NHR radical where R is alkyl, cycloalkyl, cycloalkylalkyl, acyl, sulfonyl, aryl, aralkyl, heteroaryl, heteroaralkyl, heterocyclyl, heterocyclylalkyl, hydroxyalkyl, alkoxyalkyl, or aminoalkyl, each as defined herein, *e.g.,* methylamino, 2-phenylamino, hydroxyethylamino, and the like. When R is alkyl, the group is referred to herein as monoalkylamino.

A "pharmaceutically acceptable salt" of a compound means a salt that is pharmaceutically acceptable and that possesses the desired pharmacological activity of the parent compound. Such salts include: acid addition salts, formed with inorganic acids such as hydrochloric acid, hydrobromic acid, sulfuric acid, nitric acid, phosphoric acid, and the like; or formed with organic acids such as formic acid, acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4- hydroxybenzoyl)benzoic acid, cinnamic acid, mandelic acid, methanesulfonic acid, ethanesulfonic acid, 1 ,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, glucoheptonic acid, 4,4'-methylenebis-(3-hydroxy-2-ene-I-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or salts formed when an acidic proton present in the parent compound either is replaced by a metal ion, *e.g.,* an alkali metal ion, an alkaline earth ion, or an aluminum ion; or coordinates with an organic base such as ethanolamine, diethanolamine, triethanolamine, tromethamine, N-methylglucamine, and the like. It is understood that the pharmaceutically acceptable salts are non-toxic. Additional information on suitable pharmaceutically acceptable salts can be found in Remington's Pharmaceutical Sciences, 17th ed., Mack Publishing Company, Easton, PA, 1985. The compounds of the present disclosure can also exist as cocrystals.

The compounds of the present disclosure may have asymmetric centers. Compounds of the present disclosure containing an asymmetrically substituted atom may be isolated in optically active, racemic forms or other mixtures of isomers. It is well known in the art how to prepare optically active forms, such as by resolution of materials. All chiral, diastereomeric, racemic forms are within the scope of this disclosure, unless the specific stereochemistry or isomeric form is specifically indicated.

Certain compounds of can exist as tautomers and/or geometric isomers. All possible tautomers and cis and trans isomers, as individual forms and mixtures thereof are within the scope of this disclosure. Additionally, as used herein the term alkyl includes all the possible isomeric forms of said alkyl group albeit only a few examples are set forth.

A "pharmaceutically acceptable carrier or excipient" means a carrier or an excipient that is useful in preparing a pharmaceutical composition that is generally safe, non-toxic and neither biologically nor otherwise undesirable, and includes a carrier or an excipient that is acceptable for veterinary use as well as human pharmaceutical use. "A pharmaceutically acceptable carrier/excipient" as used in the specification and claims includes both one and more than one such excipient.

"Sulfonyl" means a -SO₂R radical where R is alkyl, haloalkyl, aryl, aralkyl, heteroaryl, heteroaralkyl, heterocyclyl, heterocyclylalkyl, each as defined herein, *e.g.,* methylsulfonyl, phenylsulfonyl, benzylsulfonyl, pyridinylsulfonyl, and the like. When R is alkyl, it is also referred to herein as alkylsulfonyl.

"Substituted alkyl" means a linear saturated monovalent hydrocarbon radical of one to six carbon atoms or a branched saturated monovalent hydrocarbon radical of three to six carbon atoms where one or two hydrogen atoms in the alkyl chain are independently replaced by hydroxyl, halo, alkoxy, amino, monosubstituted amino, disubstituted amino, cyano, sulfonyl, aminocarbonyl, aminosulfonyl, -NHCONH₂, carboxy, acyl, acylamino, phenyl, or alkoxycarbonyl, each group as defined herein.

"Substituted alkynyl" means a linear saturated monovalent hydrocarbon radical of two to six carbon atoms or a branched monovalent hydrocarbon radical of three to six carbon atoms containing a triple bond where one or two hydrogen atoms in the alkynyl chain are independently replaced by phenyl, hydroxyl, alkoxy, amino, monosubstituted amino, disubstituted amino, cyano, sulfonyl, aminocarbonyl, aminosulfonyl, - NHCONH₂, carboxy, acyl, acylamino, or alkoxycarbonyl, each group as defined herein.

"Treating" or "treatment" of a disease includes: preventing the disease, i.e. causing the clinical symptoms of the disease not to develop in a mammal that may be exposed to or predisposed to the disease but does not yet experience or display symptoms of the disease; inhibiting the disease, *i.e.,* arresting or reducing the development of the disease or its clinical symptoms; or relieving the disease, *i.e.,* causing regression of the disease or its clinical symptoms.

### III. Exemplary Embodiments

### a. Histone Demethylase Inhibitors

The Jumonji family of histone demethylases has been the focus of much study over the last few years. Numerous reports have demonstrated the relevance of these enzymes in a variety of physiological and pathological conditions beyond cancer, including early development, reproduction, metabolism, and cardiac hypertrophy. The structure of Jumonji catalytic domains shows they are drugable, able to accommodate small molecule disruptors, making them ideal molecular targets for intervention. Modulation of aberrant Jumonji demethylase activity in disease should lead to the normalization of transcriptional patterns, such as we see with JIB04 in cancer cells. JIB04's ability to block tumor growth and prolong cancer survival may involve both direct and indirect aggregate effects of Jumonji enzyme pan-inhibition in cells and *in vivo,* and it is possible that the drug accumulate in cancer cells over time increasing its effective concentration/apparent potency. Mechanistically, JIB04 appears to chelate iron in the catalytic site of Jumonji enzymes and to disrupt histone substrate binding, while not being a competitive inhibitor for α-ketoglutarate, a mechanism not yet described for Jumonji inhibitors. Future structural information will be necessary to establish the exact molecular interactions between Jumonji enzymes and their cofactors/substrates that are disrupted by JIB04. One possibility is that the inhibitor may occupy the outer portion of the active site where the iron and the histone substrate bind or bind iron in the active site in a manner that alters subsequent substrate binding. Chelation in solution may also contribute to inhibition under conditions of high free iron.

In an embodiment according to the claimed invention, the anti-PESC agent is JIB-004 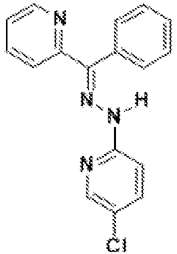 JIB04 (NSC693627, E-isomer) is a potent, selective and cell permeable Jumonji histone demethylase inhibitor. Unlike the other known inhibitors, JIB04 is not a competitive inhibitor of α-ketoglutarate. It inhibits the demethylase activity of Jumonji enzymes *in vitro,* with IC₅₀ ~230 nM for JARID1A (KDM5A), ~440 nM for JMJD2A (KDM4A) and JMJD2B (KDM4B), ~340 nM for JMJD2E (KDM2E), and ~1 µM for JMJD3 (KDM6B) and JMJD2C (KDM4C). JIB04 blocks Jumonji demethylase activity in cells and consequently inhibits cell growth, without affecting other α-ketoglutarate-dependent hydroxylases or histone-modifying enzymes, especially HDACs. JIB04 alters transcriptional programs in cancer but not in normal cells, leading to cancer-specific cell death. Importantly, *in vivo,* JIB04 lowers histone demethylase activity in tumors, reduces tumor burden and prolongs survival of mice in an aggressive breast cancer model.

Other agents that inhibit a JmjC polypeptide are mentioned herein for reference purposes and include SD-70, ML324, KDM5-C70, PBIT, KDOHP64a, KDOQZ5, IOX1, IOX2, KDOMA83, KDMOBP69, NSC636819, pyrido[3,4-pyrimidin-4(3H)-one derivatives, 3- amino-4-pyridine carboxylate derivatives or analogs thereof. See PCT WO2017190009A1.

### ML-324

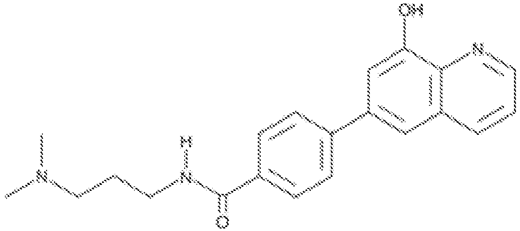

### JmjC Histone Demethylase Inhibitor, n-Octyl-IOX1

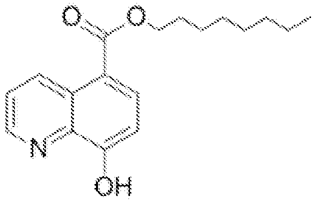

### KDM4A/KDM4B Inhibitor, NSC636819

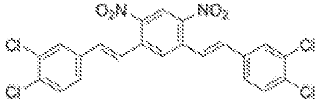

### JMJD2 Inhibitor, 5-carboxy-8HQ - CAS 5852-78-8

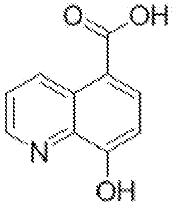

### JMJD Histone Demethylase Inhibitor III - Calbiochem

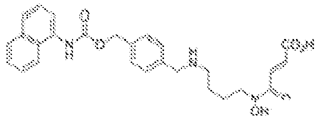

### JMJD Histone Demethylase Inhibitor IV, Methylstat - CAS 1310877-95-2 - Calbiochem

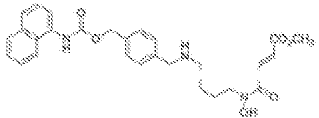

JIB04 as well as other histone demethylase inhibitors are also described in Thinnes et al. Biochimica et Biophysica Acta - Gene Regulatory Mechanisms, Volume 1839, Issue 12, December 2014, Pages 1416-1432 and Epigenetic Drug Discovery ed. Wolfgang Sippl, Manfred Jung, Raimund Mannhold, Helmut Buschmann, Jörg Holenz, John Wiley & Sons, Feb 11, 2019 ISBN: 978-3-527-34314-0.

In one reference-embodiment, the JMJD3 demethylase inhibitor comprises ethyl 3-((6-(4,5-dihydro-1H-benzo[d]azepin-3(2H)-yl)-2-(pyridin-2-yl)pyrimidin-4-yl)amino)propanoate (GSK-J4), and active derivatives thereof as disclosed in Kruidenier et al., "A Selective Jumonji H3K27 Demethylase Inhibitor Modulates Proinflammatory Macrophage Response," Nature 488:404-408 (2012).

GSK-J4 has the structure of:

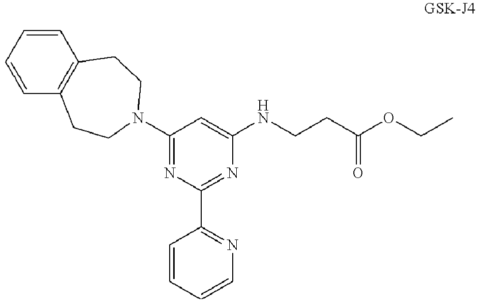

Exemplary active derivatives of GSK4 that are also JMJD3 demthylase inhibitors include GSK-J1 and GSK-J3, which have the following structures:

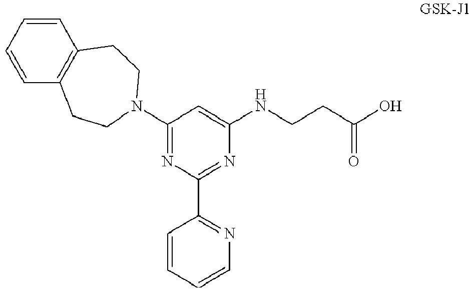

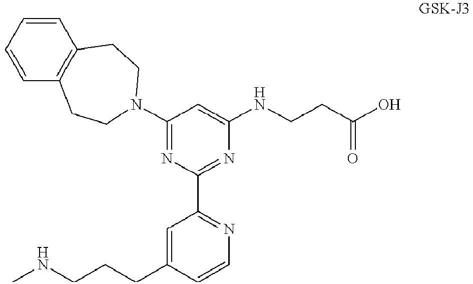

In another reference-embodiment, the JMJD3 demethylase inhibitor comprises a modified GSK-J1 small molecule as described by Hu et al., "Design and Discovery of New Pyrimidine Coupled Nitrogen Aromatic Rings as Chelating Groups of JMJD3 Inhibitors," Bioorg. Med. Chem. Lett. 26(3):721-725 (2016).

### c. Proteasome Inhibitors

In another embodiment according to the claimed invention, the anti-PESC agent is a proteasome inhibitor which is delanzomib (also known as CEP-18770).

Further proteasome inhibitors described herein for reference purposes include (a) peptide boronates, such as bortezomib (also known as Velcade^{™} and PS341), ixazomib(also known as MLN9708) or ixazomib citrate; (b) peptide aldehydes, such as MG132 (Z-Leu-Leu-Leu-H), MG115 (Z-Leu-Leu-Nva-H), IPSI 001, fellutamide B, ALLN (Ac-Leu-Leu-N1e-H, also referred to as calpain inhibitor I), and leupeptin (Ac-Leu-Leu-Arg-al); (c) peptide vinyl sulfones, (d) epoxyketones, such as epoxomicin, oprozomib (also referred to as PR-047 or ONX 0912), PR-957 (also known as ONX 0914), and carfilzomib (also referred to as PR-171); and (e) β-lactones, such as lactacystin, omuralide, salinosporamide A (also known as NPI-0052 and marizomib), salinosporamide B, belactosines, cinnabaramides, polyphenols, TMC-95, and PS-519.

### IV. Combination Therapies - ESO Regenerative agent

According to the claimed invention, the anti-PESC agent is administered conjointly with one or more agents that selectively promote proliferation or other regenerative and wound healing activities of normal epithelial stem cells. Conjoint administration of these "ESO Regenerative agents" may be accomplished by administration of a single co-formulation, by simultaneous administration or by administration at separate times. According to the present invention, this ESO Regenerative agent is ponatinib.

Also according to the claimed invention, the anti-PESC agent is administered conjointly with one or more agents that selectively promote proliferation or other regenerative and wound healing activities of normal esophageal stem cells. Conjoint administration of these "esophageal ESO Regenerative agents" may be accomplished by administration of a single co-formulation, by simultaneous administration or by administration at separate times. According to the present invention, this ESO Regenerative agent is ponatinib.

### a. ABL kinase inhibitor

Ponatinib is a pan-inhibitor of ABL kinase inhibitor, more specifically a BCR-ABL kinase inhibitor.

### b. FLT3 Inhibitors

In certain reference embodiments, the ESO Regenerative agent is a FLT3 inhibitor. Exemplary FLT3 inhibitors to be used herein are quizartinib (AC220), crenolanib (CP-868596), midostaurin (PKC-412), lestaurtinib (CEP-701), 4SC-203, TTT-3002, sorafenib (Bay-43-0006), Ponatinib (AP-24534), sunitinib (SU-11248), and/or tandutinib (MLN-0518), or (a) pharmaceutically acceptable salt(s), solvate(s), and/or hydrate(s) thereof.

Ponatinib is described in more detail below.
Brand Name: Ponatinib
Code Name: AP-24534 Structure: 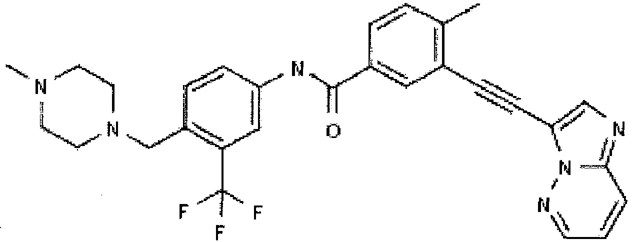
IUPAC Name: 3-[2-(Imidazo[l,2-b]pyridazin-3-yl)ethynyl]-4-methyl-N-[4-(4-methylpiperazin-I-ylmethyl)-3-(trifluoromethyl)phenyl]benzamide
Affinities: BCR-ABL, FLT3, KIT, FGFR1, PDGFRa (Gozgit, Mol Cancer Ther. 2011;10(6):1028-35).
Clinical Phase: Phase II (AML)
Developer: Ariad Pharmaceuticals (originator)

Assays for screening potential FLT3 inhibitors and, in particular, for identifying FLT3 inhibitors as defined herein, comprise, for example, *in vitro* competition binding assays to quantitatively measure interactions between test compounds and recombinantly expressed kinases¹ (Fabian et al; Nat Biotechnol. 2005 23(3):329-36). Hereby, competition with immobilized capture compounds and free test compounds is performed. Test compounds that bind the kinase active site will reduce the amount of kinase captured on solid support, whereas test molecules that do not bind the kinase have no effect on the amount of kinase captured on the solid support. Furthermore, inhibitor selectivity can also be assessed in parallel enzymatic assays for a set of recombinant protein kinases.^{2,3} (Davies et al., Biochem. J. 2000 35(1): 95-105; Bain et al. Biochem. J. 2003 37(1): 199-204). These assays are based on the measurement of the inhibitory effect of a kinase inhibitor and determine the concentration of compound required for 50% inhibition of the protein kinases of interest. Proteomics methods are also an efficient tool to identify cellular targets of kinase inliibitors. Kinases are enriched from cellular lysates by immobilized capture compounds, so the native target spectrum of a kinase inhibitor can be determined.⁴ (Godl et al;. Proc Natl Acad Sci USA. 2003 100(26): 5434-9).

Assays for screening of potential inhibitors and, in particular, for identifying inhibitors as defined herein, are, for example, described in the following papers:
- Fabian et al., Nat Biotechnol. 2005 23(3):329-36
- Davies et al., Biochem. J. 2000 351 : 95-105.
- Bain et al., Biochem. J. 2003 371 : 199-204.
- Godl et al., Proc Natl Acad Sci USA. 2003 100(26): 15434-9.

### V. Combination Therapies - Other agents

In certain embodiments, the anti-PESC agent can be administered conjointly with one or more agents that have other beneficial local activities in esophagus. Illustrative categories and specific examples of active drugs include: (a) antitussives, such as dextromethorphan, dextromethorphan hydrobromide, noscapine, carbetapentane citrate, and chlophedianol hydrochloride; (b) antihistamines, such as chlorpheniramine maleate, phenindamine tartrate, pyrilamine maleate, doxylamine succinate, and phenyltoloxamine citrate; (c) antipyretics and analgesics such as acetaminophen, aspirin and ibuprofen; (d) antacids such as aluminum hydroxide and magnesium hydroxide, (e) anti-infective agents such as antifungals, antivirals, antiseptics and antibiotics, (f) chemotherapeutic agents.

### VII. Examples

Barrett's Esophagus (BE) is an irreversible condition that is believed to be the precancerous lesion of Esophageal Adenocarcinoma (EAC). BE originates from a unique cell population preexisting at gastroesophageal junction and possesses its own stem cells. Using a novel ground-state stem cell cloning technology, we derived patient-matched stem cell pedigree lines from BE and Esophageal epithelia. Image-based high-throughput chemical screening was developed and used to uncover a unique combination of chemicals that can specifically eradicate BE stem cells while protecting and promoting Esophageal stem cells. These effects were confirmed in a co-culture 3D model and a mouse xenograft model by co-transplanting BE and Esophageal stem cells *in vivo.* Interestingly, this drug combination was also able to eradicate stem cells in patient-matched dysplasia and cancer. It is the first time that ground-state stem cells from patient-matched BE, dysplasia, cancer and normal tissue can be cloned and expanded *in vitro* and employed to identify targeted therapeutics for not only preemptive therapies of BE but therapies targeting late-stage cancers. It is anticipated that this platform can be applied to provide the promise of developing novel strategies for chemoprevention and treatment of various types of lethal cancers.

### Cloning Barrett's Esophagus Stem Cells and Chemical Screening

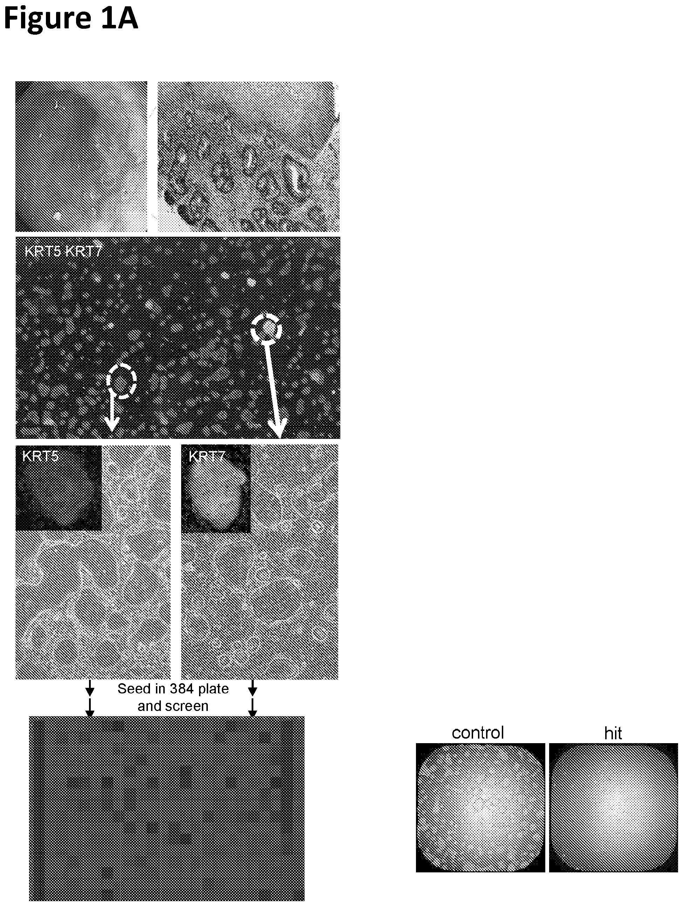

Endoscopic mucosal biopsies obtained from the distal esophagus of Barrett's patients can include Esophageal squamous epithelium and Barrett's Esophagus (Fig. 1A; Yamamoto et al). Colonies arose from single cell suspensions of these 1mm biopsies one week after plating onto lawns of irradiated 3T3 cells in growth media known to support immature, epithelial stem cells (Wang *et al.,* 2015; Yamamoto *et al.,* 2016; Duleba *et al.,* 2018) (Fig. 1A; StemECHO, MCT). As reported previously (Yamamoto *et al.,* 2016), the colonies yielded from Barrett's yielded mixtures of both Krt5-positive clones typical of the esophageal squamous basal cells and ones that expressed the columnar epithelial marker Krt7 (Fig. 1A). To separate these two populations of clones derived from the Barrett's biopsies, multiple single colonies were samples and expanded as independent pedigrees (Yamamoto *et al.,* 2016) (Fig. 1A). Reprobing these pedigree lines with the same antibodies showed that the original Barrett's biopsies contained two distinct clonogenic cells marked by committed expression of either Krt5 or Krt7 (Fig.1A).

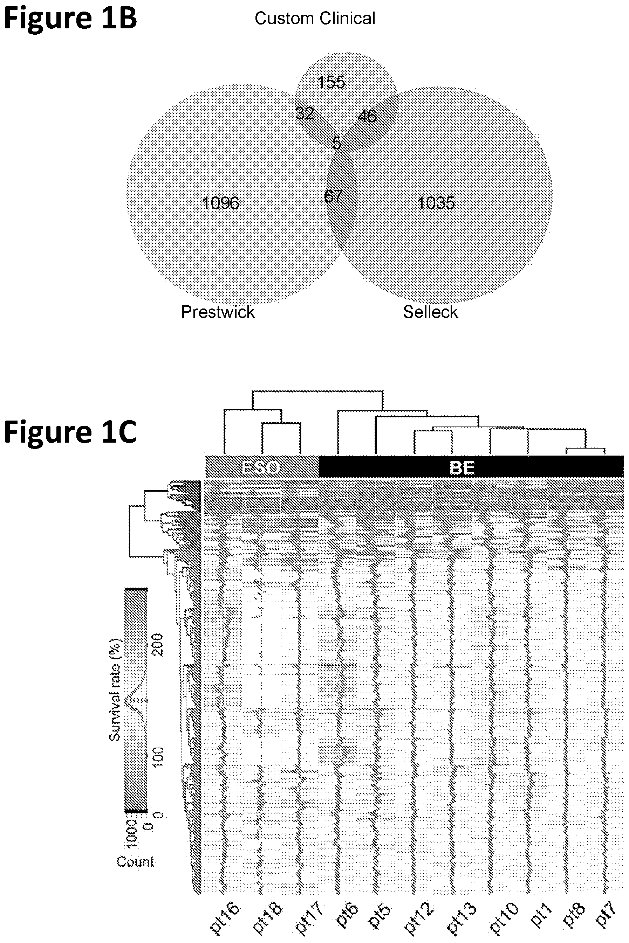

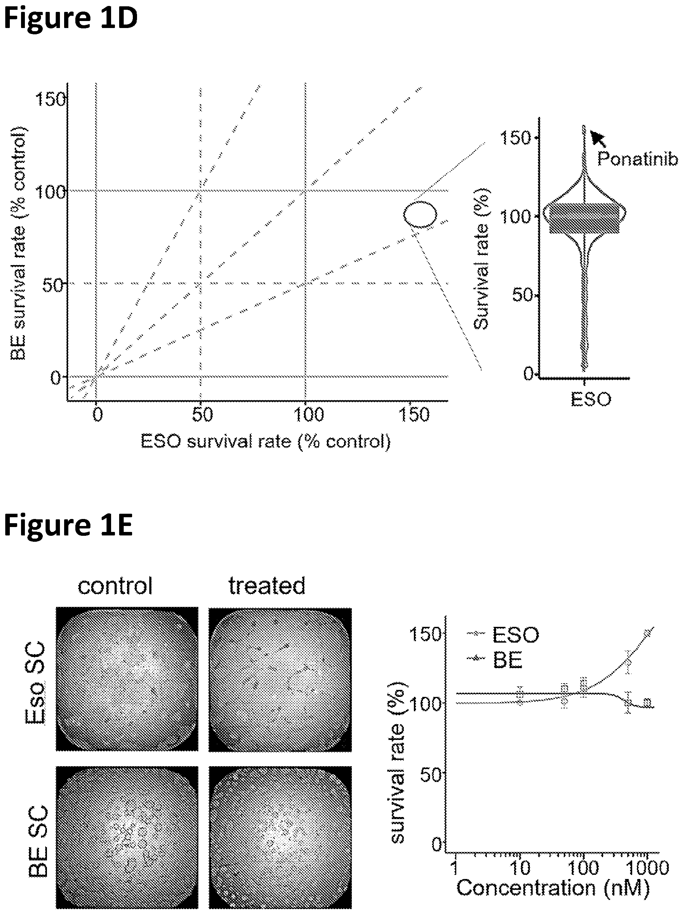

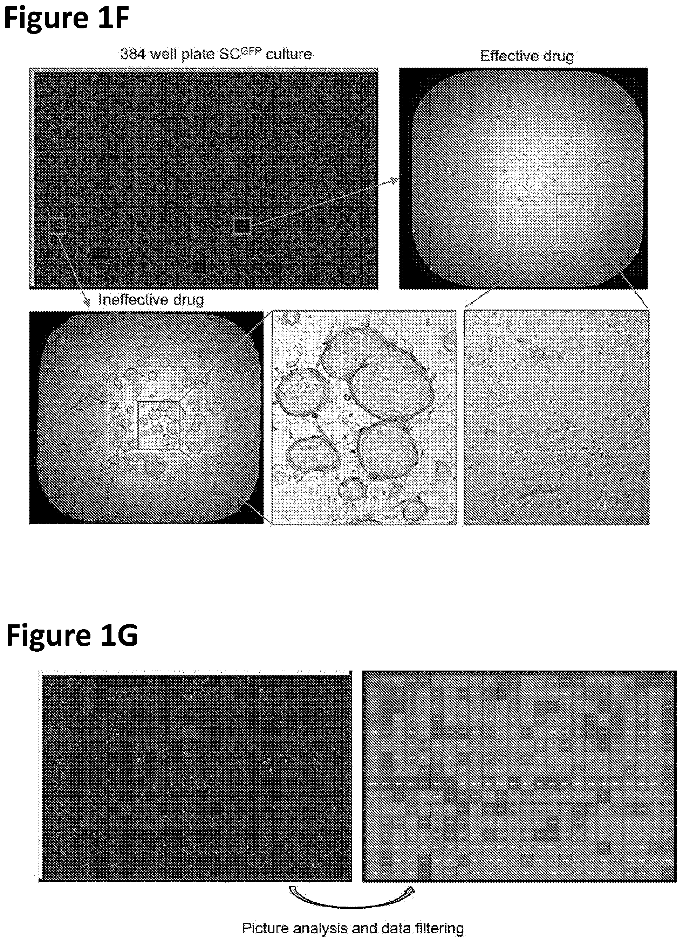

In order to explore the possibility of uncovering an agent that can selectively eradicate Barrett's stem cells, a high throughput chemical screening on a panel of esophageal squamous stem cells and Barrett's stem cells was set up. Three libraries were used for this screening including Custom Clinical, Prestwick and Selleck in totally of non-overlapping 2276 chemicals (Fig. 1B). While approximately 70% of the chemicals had no impact on either of the cell lines and around 10% of the chemicals eradicate both cell types, the other 20% of the chemicals displayed differential efforts on these two cell types (Figs. 1C, 1F and 1G). The selected hits were validated in a dosage-response manner, suggesting the reliability of the screening approach (Figs. 1F and 1G). Among these 20% of the chemicals, we did not observe one single agent that can play dual roles including eradication of Barrett's stem cells and protection of esophageal stem cells. Interestingly, Ponatinib was identified to significantly increase the proliferation of esophageal stem cells while slightly decreasing the survival of Barrett's stem cells (Fig. 1D and E).

### Synthetic Chemical Screening for Dual Action Regime

It was next hypothesized that one chemical in our libraries could function with Ponatinib in a synergistic manner to specifically eradicate Barrett's stem cells while protecting and promoting esophageal stem cells. The same libraries were screened in the background of 1µM Ponatinib. Interestingly, eight compounds displayed the lethality towards Barrett's stem cells without affecting Esophageal stem cells in the presence of Ponatinib (Fig. 2A). Among them, Adefovir Dipivoxil is a FDA approved drug, CEP-18770 and Qizartinib are in clinical trials. JIB04, WZ8040, Stattic, Nanchangmycin and AZD1080 are not in any clinical trials (Fig. 2B). None of these chemicals have been reported to target BE and interestingly, they are targeting various different pathways and harbor distinct chemical structures. Their differential effects on Barrett's stem cells and Esophageal stem cells were validated in a dosage dependent manner (Fig. 2C; Fig. 3F). Moreover, co-culture of nine Barrett's stem cell lines from patients with spectrum of mutation profiles (Yamamoto et al, 2016) and Esophageal stem cells demonstrated CEP-18770, JIB04, in the presence of Ponatinib displayed uniform lethality to all Barrett's stem cells that we tested while promoting Esophageal squamous stem cells (Fig. 2D). This result suggests that these two chemicals could work synergistically with Ponatinib to target Barrett's stem cells specifically independent of the genomics and stage of these Barrett's stem cells. The combination of Nanchangmycin and Ponatinib was not pursued due to the high lethality of 3T3-J2 feeder cells during the treatment, which suggests potential toxicity towards other types of somatic cells *in vivo.*

### Validating Dual Action Regime in 3D culture and Mouse Xenograft Model

A 3D culture model was generated to mimic Barrett's Esophagus *in vitro.* The esophageal squamous stem cells and Barrett's stem cells were co-cultured in the transwell insert. Following the creation of air-liquid interface, it was observed that the BE islands located among well-differentiated squamous epithelium that well recapitulated the histology of human BE in the patient. Given the similarity of this artificial model and human pathology, the hits were tested in this system to further validate them. Eight Barrett's stem cell lines were co-cultured with Esophageal stem cells in ALI system and CEP-18770 or JIB04 together with Ponatinib were added in the medium following the creation of ALI. The esophageal squamous stem cells were labeled with red fluorescent protein (RFP) while Barrett's stem cells were labeled with green fluorescent protein (GFP). Without the treatment, it was observed that a mixture of GFP and RFP labeled cells in culture. However, following the treatment of either CEP-18770 or JIB04 in the presence of Ponatinib, the disappearance of GFP labeled Barrett's stem cells was observed. The cross sections of the ALI structures showed intact esophageal squamous epithelium without any existence of Barrett's epithelium (Fig. 2 A&B).

The effect of these chemicals on BE in a xenograft model was further examined. The esophageal squamous stem cells and GFP labeled Barrett's stem cells were mixed and co-injected into the immunodeficient mice. After five days of xenograft, the Ponatinib and CEP-18770 combination or Ponatinib and JIB04 combination was injected into the mice intraperitoneally in an alternative manner (Fig. 3C). Two weeks following the treatment, the xenografts were collected and examined through histology analysis and clonogenic analysis. It was found that the total loss of Barrett's structures in the treated animals, which is consistent with the lack of clonogenic Barrett's stem cells *in vitro* (Fig. 3D and 3E; Fig. 4E ; Figs. 5F & 5G).

### Stem Cells of Barrett's Esophagus, Dysplasia and Cancer Can Be Targeted Similarly

BE has been known to be the precursor of the dysplastic and cancerous lesions of EAC. The transcriptome analysis of patient-matched Barrett's, dysplastic and EAC stem cells revealed significantly overlapping genes and pathways in comparison to the stem cells derived from Esophagus squamous epithelium. Moreover, PCA analysis of Barrett's stem cells derived from 12 patients together with dysplasia, cancer and Esophageal squamous stem cells confirmed that the stem cells of dysplasia and cancer shared very similar gene expression with Barrett's stem cells (Fig. 4 A & B). This data suggests that the strategies developed to eradicate BE could also be used to target dysplasia and EAC which have routinely relapsed from the traditional chemotherapies. The effects of the eight drug hits in combination with Ponatinib were tested in the cell culture of co-seeding esophageal stem cells and Barrett's, dysplasia or EAC stem cells. Most of these combinations could eliminate all three entities without affecting esophageal squamous stem cells in two independent patients (Fig. 4C). Importantly, the JIB04 and CEP-18770 can function in a synergistic manner with Ponatinib to eradicate precancerous, dysplastic and cancerous stem cells of EAC in both 3D culture system and mouse xenograft models (Fig. 5). Given these three entities always co-exist in patients with EAC, our strategies of eradicating all of them could help to eliminate the chance of recurrence of EAC patients drastically.

### Discussion of Results

Despite its influential position in cancer prevention, and fundamental advances in ablative approaches aiming to eradicate it preemptively, Barrett's remains an enormous and growing problem with an estimated 3 million cases in the US alone. Advanced BE lesions that are treated by ablative therapies recur at alarming rates (reference), suggesting an unmet medical need for effective and targeted therapeutic options for these patients. The ability to clone and expand the ground-state stem cells of BE, dysplasia, EAC and normal esophageal squamous epithelium from matched patient samples provides a very promising platform for high-throughput chemical screening devoted to developing highly selective means of eradicating BE and more advanced lesions ahead of the onset of cancer. Each of these stem cell clones can be individually differentiated by polarization in so-called "air-liquid interface" cultures to yield *3-D* epithelia remarkably similar to that of the *in situ* normal, lesional or cancerous epithelia. These same stem cell clones were used in advanced co-culture models with normal epithelial stem cells to investigate the potential ability of selected drug combinations to alter the competitive status of such lesions in the distal esophagus. Furthermore, a novel mouse model for testing drug combinations *in vivo* was established by transplanting patient-derived BE, dysplasia, EAC and esophageal squamous stem cells in the NSG mice subcutaneously.

The first direction of chemical screening focused on single agents that showed a greater effect against BE stem cells than the patient-matched esophageal stem cells as that was a key goal of this effort. However, upon detailed dose-response studies of these single agent "hits", the standard differential at the optimal dosing was, at best, 10-fold in concentration. This 10-fold differential in concentration between lethal effects on BE stem cells and those of normal esophagus was disappointing in that it suggested a relatively narrow therapeutic window.

The potential of the small molecules that selectively favored the growth of the normal esophageal stem cells was explored. The intestinal metaplasia of BE is thought to be in competition with the surrounding esophageal mucosa during its proximal spread (Wang *et al.,* 2011). Thus, in any therapeutic strategy, it is possible that this competitive interaction could be exploited from the standpoint of improving the competitive edge of the esophagus. Therefore, it is hypothesized that the molecules found to promote the growth of esophageal stem cells, such as Ponatinib could be used in combination with molecules that limit BE stem cells. Established herein are "synthetic lethal" screens (McCormick, 2015; Thompson *et al.,* 2017; Aguirre and Hahn, 2018) against BE stem cells using small molecule libraries in a background of Ponatinib. Interestingly, this synthetic lethal screen identified a different set of small molecules that, in the context of Ponatinib, efficiently kill BE stem cells while at the same time augmented the growth of normal esophageal stem cells. Among them, JIB04 and CEP-18770 in a synergistic manner with Ponatinib were most effective in eradicating BE stem cells of a wide-range of patients. Most important from the standpoint of potential therapeutics, dose-response curves for these anti-BE stem cell components of this combination, in the background of a fixed concentration of the Ponatinib, shows a remarkable differential of nearly 1,000-fold.

Significant progress has been made in mimicking the competitive interactions between BE and esophageal stem cells through the generation of co-cultures *in vitro.* Thus, "squamous islands" (Sharma *et al.,* 1998) of esophageal epithelia interspersed with regions dominated by Barrett's esophagus can be generated. These steady state co-cultures have been used to test the impact of the synthetic lethal drug combinations identified in small molecule screens. Significantly, these drug combinations have proven to be remarkably effective in eliminating the Barrett's esophagus and permitting the esophageal stem cells to expand to fill in the regions vacated by the loss of the BE epithelia. Similar efforts were also made to model the competitive interactions between Barrett's and esophageal epithelia *in vivo* in xenografts. Remarkably, these two drug combinations showed effects on dysplasia and EAC stem cells with similar selectivity and doses as those towards Barrett's stem cells. The large differential between doses of these two effective combinations that eliminated BE, dysplasia, and EAC stem cells versus normal esophageal stem cells suggest the possibility that such drug combinations could produce larger "therapeutic windows" than we observed for single agents directed at the Barrett's alone.

Therefore, in addition to identifying synthetic lethal drug combinations effective for BE, these compounds are shown to also be effective for targeting progenitor cells of dysplasia and EAC that were not part of the initial screen. This finding raises the question as to whether these compounds are targeting some feature of the Correa sequence "lineage" rather than traditional chemotherapeutics that target DNA synthesis or common activities of a rapidly dividing cell.

Taken together, precancerous and cancerous lesions are regenerative and like normal regenerative epithelia depend on discrete populations of immature stem cells for this regenerative growth, and that therapeutics directed at these stem cell populations could be highly specific and effective approaches to eliminating these lesions for therapeutic benefit. Therefore, this platform and similar approaches can be applied to drug discovery efforts in other cancer types.

### Methods

### High throughput screening and imaging of selected cell lines

The GFP-tagged cell lines will be seeded on multiple 384 well plates (Griener Bio-One, USA) (varying according to number of compounds in a library) with a feeder layer of irradiated 3T3-J2 fibroblast cells. The stem cell will be allowed to grow until they divide to become 4-5 cells within a colony. Afterwards they will be transported for treatment with selected chemical library (1 µM) to High Throughput Research and Screening Center at Institute of Biosciences and Technology (Houston, Texas), Texas A&M University. Positive and negative control lanes will be allocated within each plate. A highly potent drug will be used as a positive control and negative control will be just with DMSO since most of the drugs are dissolved in DMSO. The cells will be allowed to grow for 6 days at 37 °C, 7.5% CO2 incubator after treatment. After the cells in the control lanes are at good confluency, the cells will be prepared for imaging. Each multi-well plates will be washed with Phosphate Buffered Saline (Gibco, USA) and fixed with 4% paraformaldehyde at room temperature for 25 minutes. Paraformaldehyde will be replaced with Phosphate Buffered Saline (PBS) and will be imaged using Inverted Eclipse Ti-Series (Nikon, Japan) microscope with Lumencor SOLA light engine, paired with High Content Microscope system (Nikon, Japan), Andor Technology Clara Interline CCD camera, NIS-Elements Advanced Research v.4.13 software (Nikon, Japan) and NIS-Elements HC software (Nikon, Japan). High Content Analysis (HCA) system built on NIS-Elements platform streamlined with automated well plate acquisition and multiple well plate job run will be used for high throughput imaging of phase contrast as well as FITC channel.

### High Content Image Analysis, selection of drugs, and dose-response analysis

NIS-Elements High Content Analysis (HCA) system will be used for image data management of multiple well plate job runs. The cells labelled with Green Fluorescent Protein (GFP) in each well of multi well plates will be imaged with features of each stem cell colonies. The changes in cell phenotype compared to control lanes will be measured based on the fluorescent signal threshold using automated image analysis. The features like area, colony number will be exported from the automated analysis. Treated wells will be compared to untreated wells based on GFP-signal area and number of stem cells colonies. The treated well will be normalized with the untreated well based on the area which will be represented in terms of survival rate (percentage of control¹) and will be compared between control and pathogenic population of cells. Z'-factor^{2,3} will be calculated based on the difference between positive and negative control and will be used as criteria for assessing quality of runs. Only plates with Z'-factor >0.6 will be used. The compounds with a coefficient of variation between plate duplicates larger than 20% will be ignored. B-score¹ will be calculated via R package platetools v0.0.2 (github.com/swarchal/platetools) to control the edge and positional bias to help infer the potential hits of inhibitors. Only the compounds with a cutoff of B-score < -2 will be considered as potential inhibitors. The selection of drugs will be made based on the maximum differences in survival rate between patient-matched cell lines (cut-off set at 20%), their targets, structural spectrum, pathways related to them and their possible relation implicated in Barret's esophageal. If there are not patient matched pedigrees, the median value of survival rates in a contain group will be used as representative value for that group of pedigrees. The dose-response curves of survival rate for a certain compound will be calculated by fitting a three-parameter log-logistic dose-response model to the survival rate data using R package *drc⁴* v3.0.1. R packages *Imtesf⁵* v0.9-36 and *sandwich⁶* v2.4-0 to obtain robust standard errors to address the fact that some variance heterogeneity is present. The ED50 value is estimated by module ED in the R package *drc.*

### VIII. References

1. Malo, N., Hanley, J.A., Cerquozzi, S., Pelletier, J. & Nadon, R. Statistical practice in high-throughput screening data analysis. Nat Biotechnol 24, 167-75 (2006).
2. Zhang, J.H., Chung, T.D. & Oldenburg, K.R. A Simple Statistical Parameter for Use in Evaluation and Validation of High Throughput Screening Assays. J Biomol Screen 4, 67-73 (1999).
3. Iversen, P.W., Eastwood, B.J., Sittampalam, G.S. & Cox, K.L. A comparison of assay performance measures in screening assays: signal window, Z' factor, and assay variability ratio. J Biomol Screen 11, 247-52 (2006).
4. Ritz, C., Baty, F., Streibig, J.C. & Gerhard, D. Dose-Response Analysis Using R. Plos One 10(2015).
5. Zeileis, A. & Hothorn, T. Diagnostic Checking in Regression Relationships. R News 2, 7-10 (2002).
6. Zeileis, A. Econometric Computing with HC and HAC Covariance Matrix Estimators. Journal of Statistical Software 1, 1-17 (2004).

## Claims

1. A drug eluting device for use as part of a treatment for one or more of chronic inflammatory injury, metaplasia, dysplasia or cancer of an epithelial tissue, which device comprises drug release means including:
(a) ponatinib; and
(b) an anti-PESC agent selected from the group consisting of 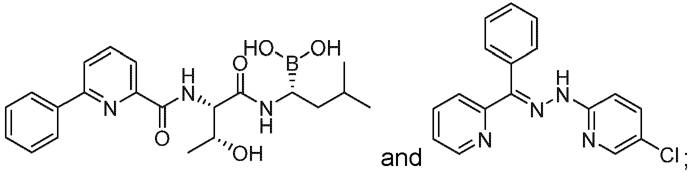 which device when deployed in a patient positions the drug release means proximal to the surface of the diseased tissue and releases ponatinib and the anti-PESC agent in an amount sufficient to achieve a therapeutically effective exposure of the diseased tissue to the ponatinib and the anti-PESC agent.

2. The drug eluting device of claim 1, for treating one or more of esophagitis, Barrett's esophagus, esophageal dysplasia or esophageal cancer.

3. The drug eluting device of claim 1, wherein the drug eluting device is a drug eluting stent, or a balloon catheter having a surface coating including the agent.

4. The drug eluting device of any of the preceding claims, further comprising one or more antitussives, antihistamines, antipyretics, analgesics, anti-infective agents and/or chemotherapeutic agents.

5. The drug eluting device of any of the preceding claims, wherein the ponatinib and anti-PESC agent are:
(a) administered as separate formulations; or
(b) co-formulated together.

6. The drug eluting device of claim 4, wherein the one or more antitussives, antihistamines, antipyretics, analgesics, anti-infective agents and/or chemotherapeutic agents are co-formulated together with the ponatinib and anti-PESC agent.

## Patentansprüche

1. Vorrichtung zur Freisetzung von Arzneimitteln zur Verwendung als Teil einer Behandlung für eine oder mehrere chronische entzündliche Verletzungen, Metaplasie, Dysplasie oder Krebs eines Epithelgewebes, wobei die Vorrichtung Mittel zur Freisetzung von Arzneimitteln umfasst, einschließlich:
(a) Ponatinib; und
(b) ein Anti-PESC-Mittel, ausgewählt aus der Gruppe, bestehend aus 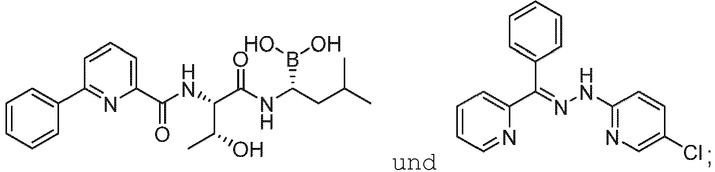 wobei die Vorrichtung, wenn sie bei einem Patienten eingesetzt wird, das Mittel zur Freisetzung des Arzneimittels proximal zur Oberfläche des erkrankten Gewebes positioniert und Ponatinib und das Anti-PESC-Mittel in einer Menge freisetzt, die ausreicht, um eine therapeutisch wirksame Exposition des erkrankten Gewebes gegenüber dem Ponatinib und dem Anti-PESC-Mittel zu erreichen.

2. Vorrichtung zur Freisetzung von Arzneimitteln nach Anspruch 1 zur Behandlung von Ösophagitis, Barrett-Ösophagus, Ösophagusdysplasie oder Ösophaguskrebs.

3. Vorrichtung zur Freisetzung von Arzneimitteln nach Anspruch 1, wobei die Vorrichtung zur Freisetzung von Arzneimitteln ein Stent oder ein Ballonkatheter mit einer Oberflächenbeschichtung ist, die das Mittel einschließt.

4. Vorrichtung zur Freisetzung von Arzneimitteln nach einem der vorstehenden Ansprüche, ferner umfassend ein oder mehrere Antitussiva, Antihistaminika, Antipyretika, Analgetika, antiinfektiöse Mittel und/oder chemotherapeutische Mittel.

5. Vorrichtung zur Freisetzung von Arzneimitteln nach einem der vorstehenden Ansprüche, wobei das Ponatinib und das Anti-PESC-Mittel:
(a) als getrennte Formulierungen verabreicht werden; oder
(b) gemeinsam formuliert werden.

6. Vorrichtung zur Freisetzung von Arzneimitteln nach Anspruch 4, wobei ein oder mehrere Antitussiva, Antihistaminika, Antipyretika, Analgetika, antiinfektiöse Mittel und/oder chemotherapeutische Mittel zusammen mit dem Ponatinib und dem Anti-PESC-Mittel formuliert sind.

## Revendications

1. Dispositif d'élution de médicament destiné à être utilisé dans le cadre d'un traitement de l'un ou plusieurs parmi une lésion inflammatoire chronique, une métaplasie, une dysplasie ou un cancer d'un tissu épithélial, lequel dispositif comprend des moyens de libération de médicament comportant :
(a) du ponatinib ; et
(b) un agent anti-PESC choisi dans le groupe constitué de 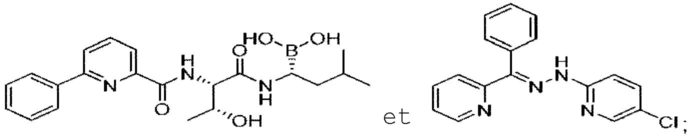 lequel dispositif, lorsqu'il est déployé chez un patient, positionne les moyens de libération de médicament à proximité de la surface du tissu malade et libère le ponatinib et l'agent anti-PESC en une quantité suffisante pour obtenir une exposition thérapeutiquement efficace du tissu malade au ponatinib et à l'agent anti-PESC.

2. Dispositif d'élution de médicament selon la revendication 1, pour traiter une ou plusieurs maladies parmi l'œsophagite, l'œsophage de Barrett, la dysplasie œsophagienne ou le cancer de œsophage.

3. Dispositif d'élution de médicament selon la revendication **1,** dans lequel le dispositif d'élution de médicament est un stent **d'élution** de médicament ou un cathéter à ballonnet ayant un revêtement de surface comportant l'agent.

4. Dispositif d'élution de médicament selon l'une quelconque des revendications précédentes, comprenant en outre un ou plusieurs antitussifs, antihistaminiques, antipyrétiques, analgésiques, agents anti-infectieux et/ou agents chimiothérapeutiques.

5. Dispositif d'élution de médicament selon l'une quelconque des revendications précédentes, dans lequel le ponatinib et l'agent anti-PESC sont :
(a) administrés sous forme de formulations séparées ; ou
(b) co-formulés ensemble.

6. Dispositif d'élution de médicament selon la revendication **4,** dans lequel les un ou plusieurs antitussifs, antihistaminiques, antipyrétiques, analgésiques, agents anti-infectieux et/ou agents chimiothérapeutiques sont co-formulés avec le ponatinib et l'agent anti-PESC.
